# EUROPEAN PATENT APPLICATION

(11) **EP 3 659 435 A1**
(43) Date of publication of application: **03.06.2020**
(21) Application number: 19198486.3
(22) Date of filing: 26.05.2010
(51) Int. Cl.: A01K 67/027, A61K 35/28, A61K 48/00, C07K 14/00, C07K 14/16, C07K 14/47, C12N 9/90, C12N 15/85, C12N 15/867, C12N 15/113

(54) **DUAL VECTOR FOR INHIBITION OF HUMAN IMMUNODEFICIENCY VIRUS**

(30) Priority: 15.07.2009 US 22568709 P; 17.12.2009 US 28759909 P
(62) Divisional of application: 17197440.5
(71) Applicant: Calimmune, Inc., Tucson, AZ 85711 (US); The Regents of the University of California, Oakland, CA 94607-5200 (US)
(72) Inventor: BRETON, Louis Randall, Tucson, AZ 85711 (US); CHEN, Irvin, Tucson, AZ 85711 (US); AN, Dong Sung, Tucson, AZ 85711 (US); SYMONDS, Geoffrey P., Rose Bay, New South Wales 2029 (AU); BOYD, Maureen, Tucson, AZ 85711 (US); MILLINGTON, Michelle L., Hornsby Heights, New South Wales (AU)
(74) Representative: Mewburn Ellis LLP

(57) **Abstract**

The present invention provides an expression vector for preventing or inhibiting HIV entry, fusion or replication in mammalian cells. In particular, the invention provides a recombinant retroviral vector that encodes an inhibitor of a HIV co-receptor, such as CCR5 or CXCR4, and a protein that inhibits HIV fusion to target cells and/or HIV replication. Pharmaceutical compositions comprising such constructs and methods of use thereof to prevent or treat HIV infection in a patient are also disclosed.

## Description

### CROSS-REFERENCE TO RELATED APPLICATIONS

This application claims the benefit of priority of U.S. Provisional Application No. 61/287,599, filed December 17, 2009, and U.S. Provisional Application No. 61/225,687, filed July 15, 2009, both of which are herein incorporated by reference in their entireties.

### DESCRIPTION OF THE TEXT FILE SUBMITTED ELECTRONICALLY

The contents of the text file submitted electronically herewith are incorporated herein by reference in their entirety: A computer readable format copy of the Sequence Listing (filename: CALI_004_01WO_ScqList_ST25.txt, date recorded: May 26, 2010, file size 9 kilobytes).

### FIELD OF THE INVENTION

The present invention relates generally to the fields of molecular biology and virology.

In particular, the invention relates to expression vectors useful in the treatment and prevention of HIV infections.

### BACKGROUND OF THE INVENTION

Human immunodeficiency virus (HIV) is the causative agent of acquired immunodeficiency syndrome (AIDS) in humans, which causes the immune system to fail and increases the probability of death due to opportunistic infections. HIV infection is a major global health problem as evidenced by its designation as a pandemic by the World Health Organization. Most people who are infected with HIV eventually develop AIDS, which claims the lives of more than one million people every year.

Antiretroviral therapies, such as HAART (highly active antiretroviral therapy), which includes combinations of nucleoside analogue reverse transcriptase inhibitors, protease inhibitors, and non-nucleoside reverse transcriptase inhibitors, have dramatically decreased the morbidity and mortality rate from HIV/AIDS in regions of the world where the therapy is available. However, HAART does not cure or completely eliminate all the symptoms of HIV/AIDS. HAART is also associated with several side effects as well as the emergence of HIV strains that are resistant to the retroviral inhibitors. For these reasons as well as the high cost of HAART and need for strict adherence, such therapy can be relatively ineffective for a large number of patients. Thus, there is need in the art to develop improved strategies for treating and preventing HIV infection.

### SUMMARY OF THE INVENTION

The present invention provides a new therapeutic approach for treating and/or preventing HIV infection in which two different steps in viral infection are targeted by gene therapy. For instance, the present invention provides a vector encoding an inhibitor of viral entry into a host cell and an inhibitor of viral fusion and/or an inhibitor of viral replication. Accordingly, in one embodiment, the present invention provides an expression vector comprising a first nucleic acid sequence encoding an inhibitor of an HIV co-receptor and a second nucleic acid sequence encoding a protein that inhibits HIV fusion to the target cell or HIV replication, wherein said first nucleic acid sequence is operably linked to a first promoter and said second nucleic acid sequence is operably linked to a second promoter. The expression vector can be a viral vector, such as a retroviral or lentiviral vector. In some embodiments, the first and second nucleic acid sequences are transcribed from a single promoter. In a particular embodiment, an internal ribosome entry site (IRES) is present upstream of the second nucleic acid sequence.

In certain embodiments, the expression vector further comprises a third nucleic acid sequence encoding an inhibitor of viral entry, viral fusion, or viral replication. In some embodiments, the third nucleic acid is operably linked to a third promoter. In other embodiments, two of the three nucleic acid sequences are transcribed from a single promoter (*i.e*. the first and second nucleic acid sequences or the second and third nucleic acid sequences). In still other embodiments, all three nucleic acid sequences are transcribed from a single promoter. One or more IRES can be present upstream of the second and/or third nucleic acid sequences.

In one embodiment of the invention, the first nucleic acid sequence of the expression vector encodes an inhibitory nucleic acid molecule, such as a siRNA or shRNA, that targets an HIV co-receptor. In some embodiments, the siRNA or shRNA molecule comprises a double-stranded region having a sequence that is substantially identical and complementary to CCR5. In other embodiments, the siRNA or shRNA molecule comprises a double-stranded region having a sequence that is substantially identical and complementary to CXCR4.

In another embodiment of the invention, the second nucleic acid sequence of the expression vector encodes a protein that inhibits HIV fusion to the target cell. The HIV fusion inhibitor protein can be a C46 protein or other like proteins that inhibit fusion of HIV to the cell surges and are transgene expressed to be located on the cell surface (*e.g*., T20 and its related proteins, enfuvirtide, CP₃₂M, and sifuvirtide).

In yet another embodiment of the invention, the second nucleic acid sequence of the expression vector encodes a protein that inhibits HIV replication. For instance, in some embodiments, the second nucleic acid sequence encodes a TRIM5α protein or a derivative or fusion thereof. In certain embodiments, the second nucleic acid sequence encodes a chimeric TRTM5α in which the amino terminal domain is from a human TRIM5α protein and the carboxy terminal PRYSPRY domain is from a rhesus TRIM5α protein. In other embodiments, the second nucleic acid sequence encodes a TRIM5-cyclophilin fusion protein.

In one embodiment of the invention, the expression vector comprises a first, second, and third nucleic acid sequence, wherein the first nucleic acid sequence encodes an inhibitor of an HIV co-receptor (*e.g*., shRNA to CCR5 or CXCR4), the second nucleic acid sequence encodes a fusion inhibitor (*e.g*., C46), and the third nucleic acid sequence encodes an inhibitor of HIV replication (*e.g*., TRIM5α, protein or a derivative or fusion thereof).

In some embodiments of the invention, the inhibitor of an HIV co-receptor and the inhibitor of HIV fusion to the target cell or inhibitor of HIV replication are expressed from different promoters on the expression vector. In one embodiment, the inhibitor of an HIV co-receptor (*e.g*. CCR5 or CXCR4) is expressed from a RNA polymerase III promoter, while the inhibitor of HIV fusion and/or replication is expressed from a RNA polymerase II promoter. The two different inhibitors can be expressed in different ratios from the expression construct.

The present invention also provides methods of making the expression vectors described herein as well as pharmaceutical compositions comprising the novel expression vectors. For instance, in one embodiment, the method of producing a viral expression vector which, when present in a cell, is capable of inhibiting binding of HIV to the cell and preventing HIV fusion into the cell or HIV replication, comprises synthesizing a cDNA of a gene which expresses a protein capable of preventing HIV fusion into a cell or HIV replication; cloning the synthesized cDNA into a restriction site in a viral vector; and inserting an expression unit capable of down regulating expression of an HIV co-receptor into a restriction site in the vector.

The present invention also provides a method of treating or preventing HIV infection in a patient. In one embodiment, the method comprises administering to the patient a pharmaceutical composition comprising an expression vector of the invention. Administration of such compositions can confer resistance to infection by R5 and X4 tropic strains of HIV. In one embodiment, the patient is human. The patient may be HIV negative or HIV positive. In some embodiments, the patient may be naïve to HAART therapy, receiving HAART therapy, failing or failed on HAART therapy. In other embodiments, the patient may have full-blown AIDS (*e.g*., AIDS/lymphoma).

In another embodiment, the method comprises transducing hematopoietic cells (*e.g*., HPSC, CD4+ T lymphocytes, CD8+ T lymphocytes, or monocyte/macrophages) with an expression vector of the invention and transplanting said transduced cells in the patient, wherein said transduced cells are resistant to HIV infection. In one embodiment, the hematopoietic cells are hematopoietic progenitor/stem cells (HPSC) that generate granulocytes, monocyte/macrophages, and lymphocytes that are resistant to HIV infection following transplantation into a patient. In some embodiments, the HPSC are autologous and CD34 positive. The transduced HPSC can generate granulocytes, monocyte/macrophages, and lymphocytes that are resistant to infection by R5 and X4 tropic strains of HIV. In certain embodiments, the transduced HPSC can generate granulocytes, monocyte/macrophages, and lymphocytes that are resistant to infection by HIV strains that are resistant to HAART.

Throughout this specification, unless the context requires otherwise, the word "comprise", or variations such as "comprises" or "comprising", will be understood to imply the inclusion of a stated element, integer or step, or group of elements, integers or steps, but not the exclusion of any other element, integer or step, or group of elements, integers or steps.

Any discussion of documents, acts, materials, devices, articles or the like which has been included in the present specification is solely for the purpose of providing a context for the present invention. It is not to be taken as an admission that any or all of these matters form part of the prior art base or were common general knowledge in the field relevant to the present invention before the priority date of each claim of this specification.

In order that the present invention may be more clearly understood, preferred embodiments will be described with reference to the following drawings and examples.

### BRIEF DESCRIPTION OF THE DRAWINGS

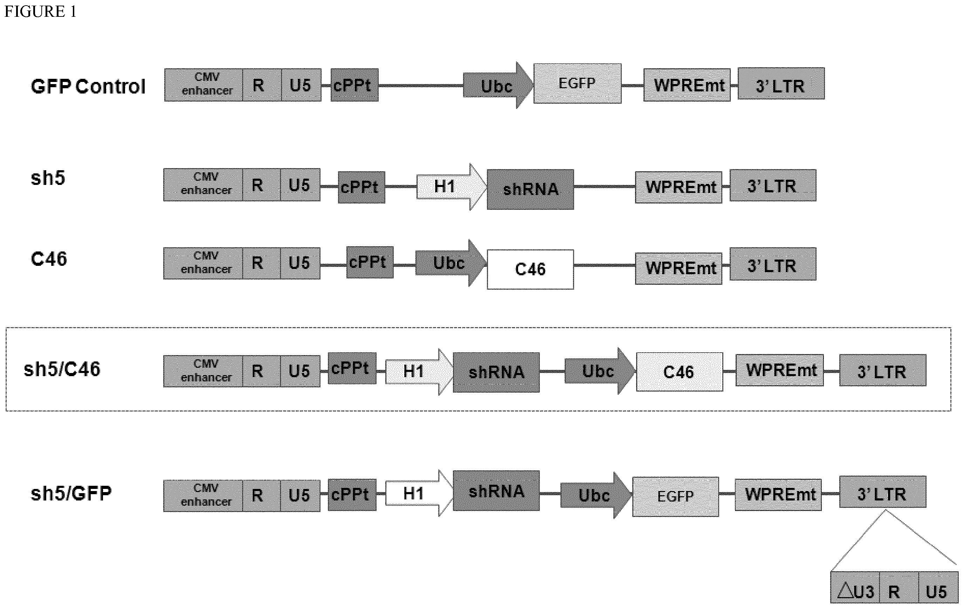

**Figure 1****. Lentiviral Vector Constructs.** Schematic showing the important elements comprising each of the indicated vectors. The dual vector sh5/C46 is shown highlighted by dotted line surround.

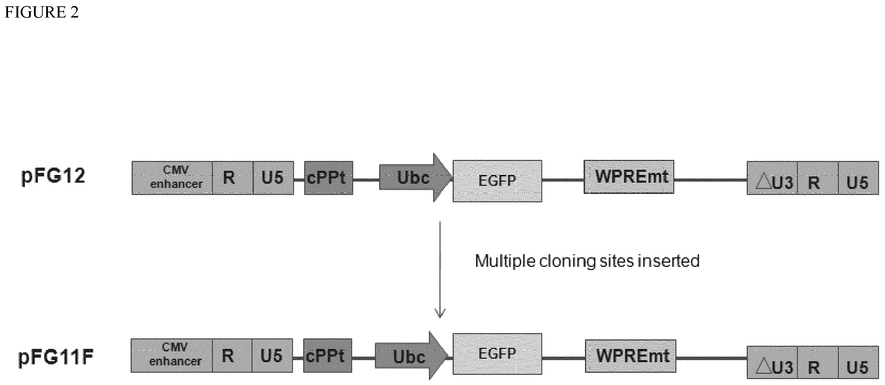

**Figure 2****. Backbone Constructs.** Schematic showing the important elements comprising each backbone lentiviral vector. p, means plasmid. pFG11F was obtained from pFG12 by inserting multiple cloning sites (MCS) at various locations including upstream of the ubiquitin promoter (Ubc).

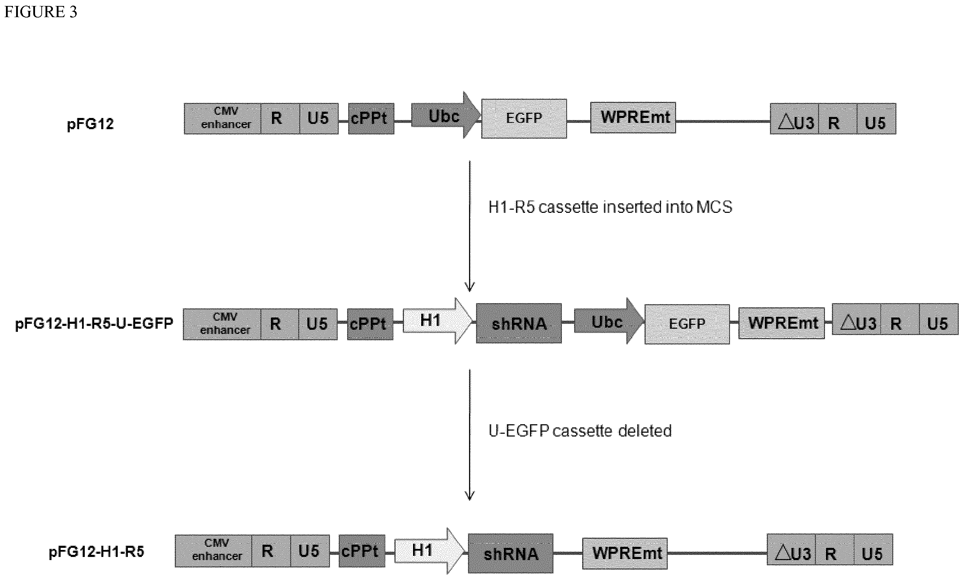

**Figure 3****. Vectors Derived from FG12 Backbone.** Schematic showing the derivation of pFG12-H1-R5-U-EGFP and pFG12-H1-R5.

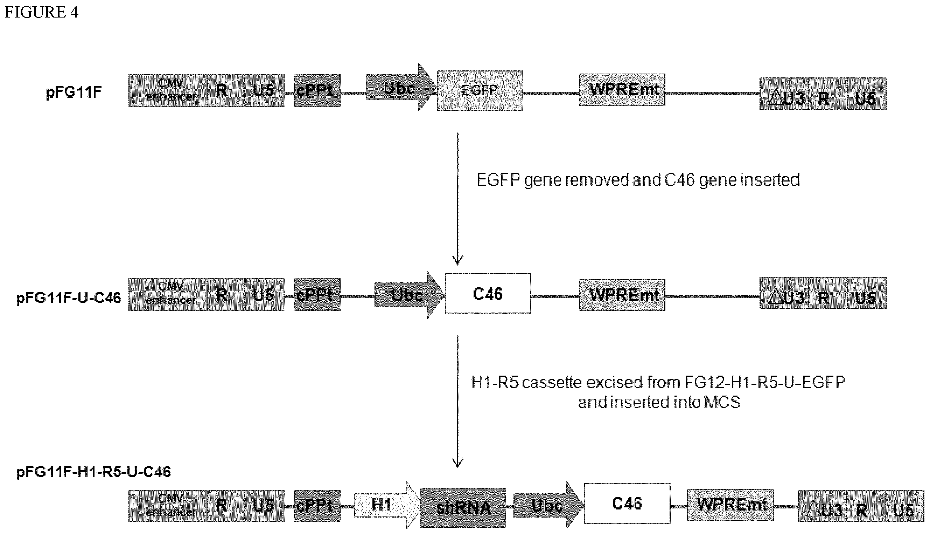

**Figure 4****. Vectors Derived from FG11F Backbone.** Schematic showing the derivation of pFG11F-U-C46 and pFG11F-H1-R5-U-C46.

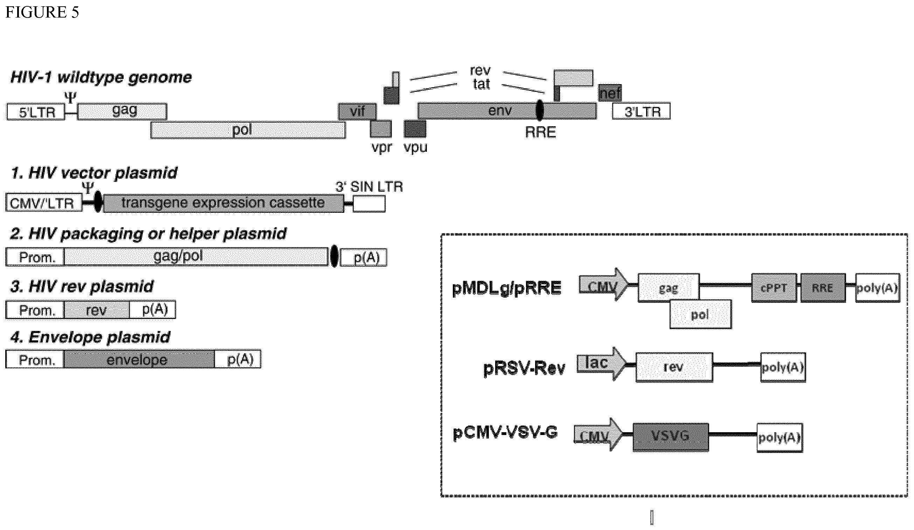

**Figure 5****. Production of Lentivirus.** Schematic (left panel) shows the HIV-1 wild-type genome and the generic vectors used in the transient co-transfection system: 1. HIV vector plasmid (test vector, *e.g.* constructs shown in Figures 1-4); 2-4 the various helper plasmids. Schematic in dashed box (right panel) shows the elements of the actual helper plasmids used in lcntiviral production.

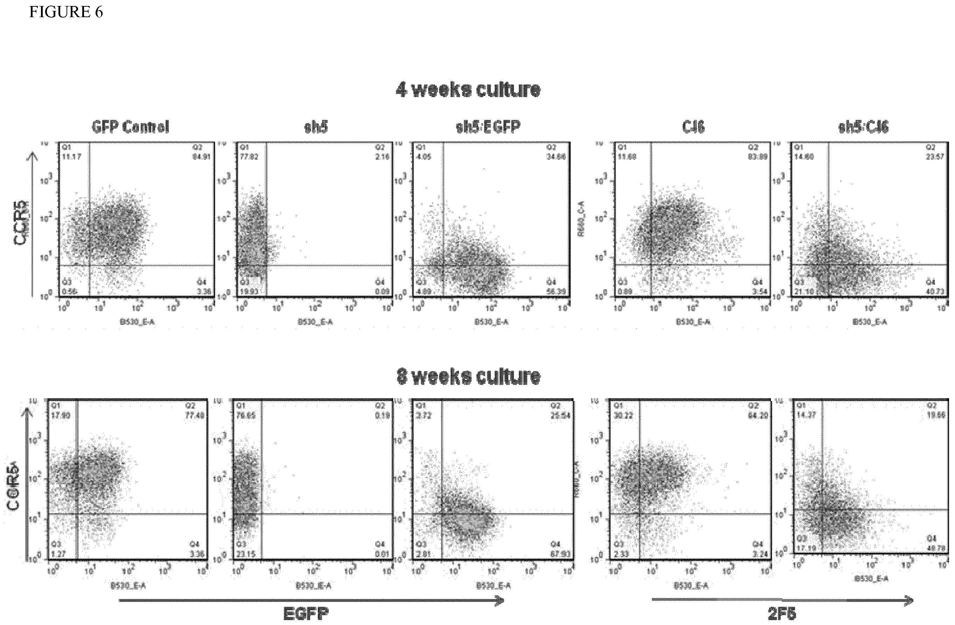

**Figure 6****. Stability of Expression in CEM.NKR.CCR5 Cells.** FACS analysis of CEM.NKR.CCR5 cells transduced with the indicated constructs at 4 and 8 weeks in culture. Cells were analyzed for CCR5 expression (via CD195 antibody), C46 expression (via 2F5 antibody), and EGFP expression. GFP expression is seen for the constructs containing EGFP (GFP control and sh5/EGFP; panels 1,3); a reduction in CCR5 expression is seen for the constructs containing sh5 (sh5, sh5/EGFP, and sh5/C46; panels 2,3,5), and C46 expression is seen for the constructs containing C46 (C46 and sh5/C46; panels 4,5). Percentage positive cells are shown in each flow cytometry quadrant (Q1-Q4). Similar results are seen at 4 and 8 weeks.

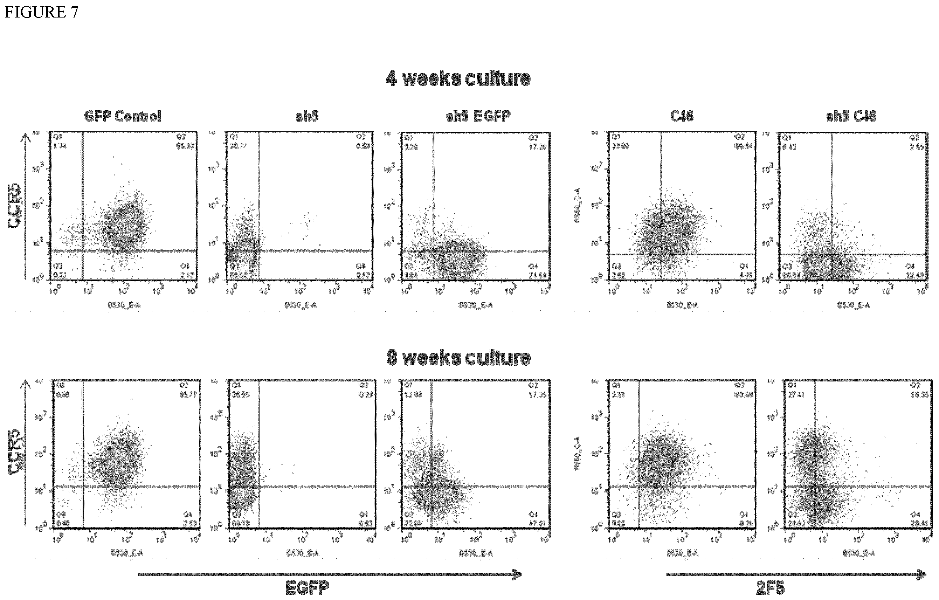

**Figure 7****. Stability of Expression in Molt4/CCR5 Cells.** FACS analysis of Molt4/CCR5 cells transduced with the indicated constructs at 4 and 8 weeks in culture. Cells were analyzed for CCR5 expression (via CD195 antibody), C46 expression (via 2F5 antibody), and EGFP expression. GFP expression is seen for the constructs containing EGFP (GFP control and sh5/EGFP; panels 1,3); a reduction in CCR5 expression is seen for the constructs containing sh5 (sh5, sh5/EGFP, and sh5/C46; panels 2,3,5), and C46 expression is seen for the constructs containing C46 (C46 and sh5/C46; panels 4,5). Percentage positive cells are shown in each flow cytometry quadrant (Q1-Q4). Similar results are seen at 4 and 8 weeks.

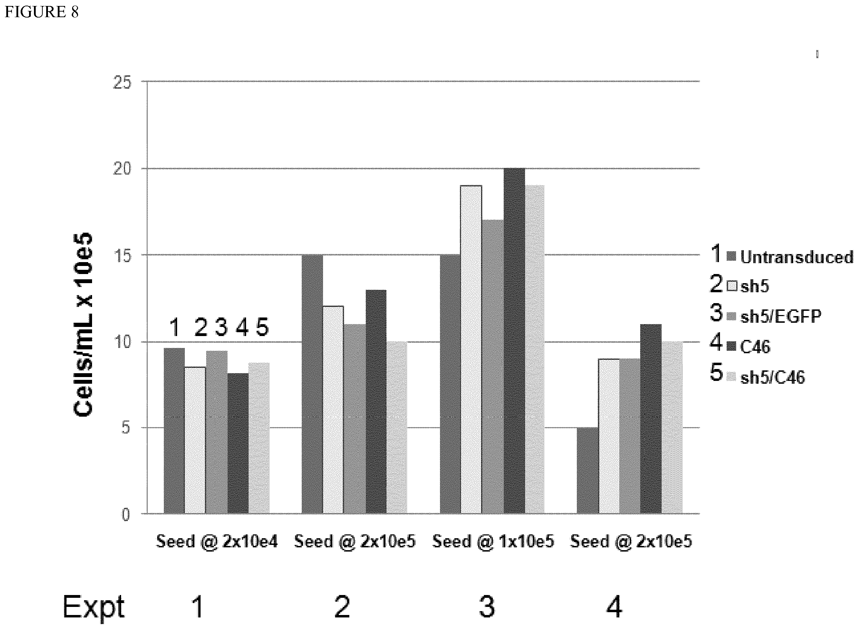

**Figure 8****. Growth Characteristics of Transduced CEM.NKR.CCR5 Cells.** Bar graph showing the number of CEM.NKR.CCR5 cells transduced with the indicated lentiviral constructs 4-7 days after seeding at the indicated concentrations; 4 independent seedings are shown numbered on X-axis 1-4. Transduction with the various constructs (sh5(2), sh5/EGFP(3), C46(4), sh5/C46(5)) had no effect on the growth rate of the cells compared to untransduced cells(1).

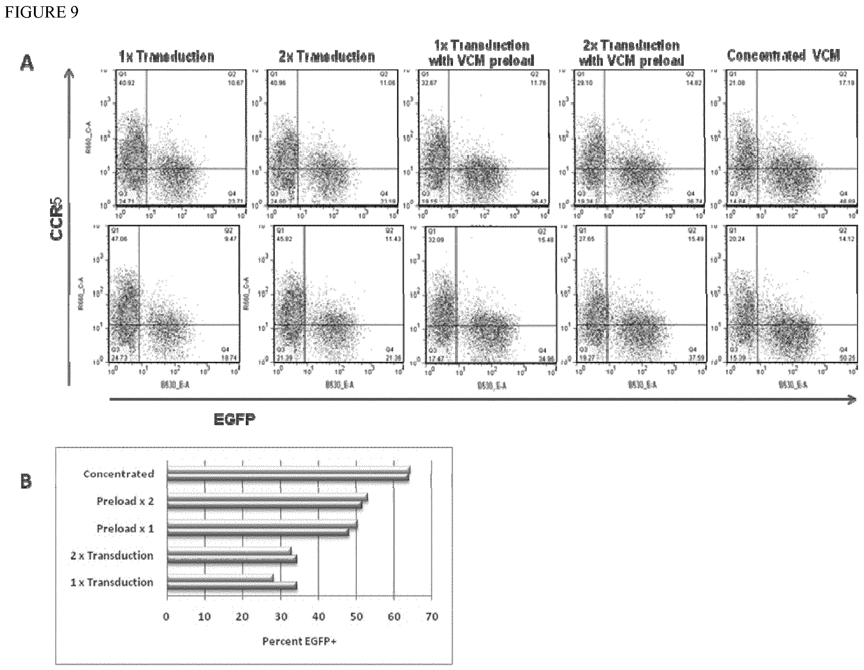

**Figure 9****. Transduction Methods for Peripheral Blood Mononuclear Cells (PBMC).** PBMC were transduced with a sh5/EGFP lentiviral construct in one of the four following ways: 1X transduction with virus containing medium (VCM), 2X transduction with VCM, 1X transduction with VCM preload (Preload x 1), 2X transduction with VCM preload (Preload x 2), and concentrated VCM. **A.** Flow cytometry analysis of PBMC transduced with sh5/EGFP lentilviral construct with the indicated methods. **B.** Summary of the percentage of EGFP positive cells for each transduction method. The results show that transduction was most efficient with concentrated virus, followed by pre-load 2X, pre-load 1X, then 2X and 1X suspension; two replicates are shown for each.

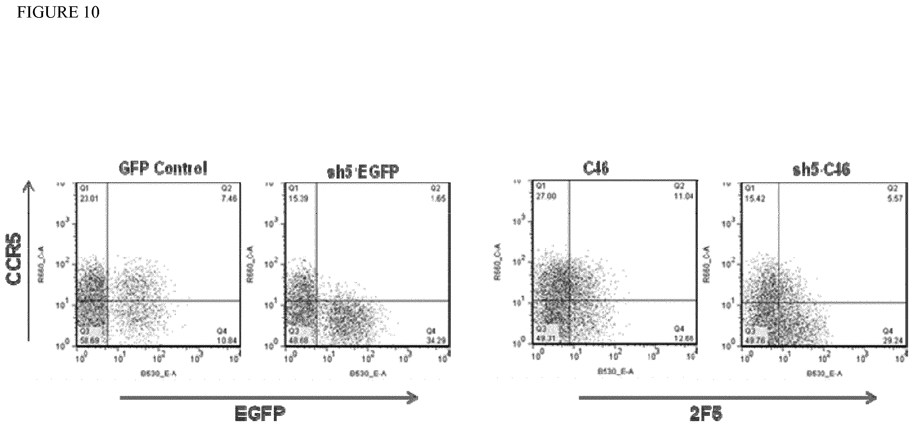

**Figure 10****. PBMC Transduction.** FACS analysis of PBMC transduced with the indicated constructs at 4 days post transduction. GFP expression is seen for the constructs containing EGFP (panels 1,2); CCR5 down-regulation is seen for the constructs containing sh5 (panels 2 and 4), and C46 expression (measured by 2F5 antibody) is seen for the constructs containing C46 (panels 3,4). MFI values, from left to right, were 16.2, 8.4, 16.8, 9.4.

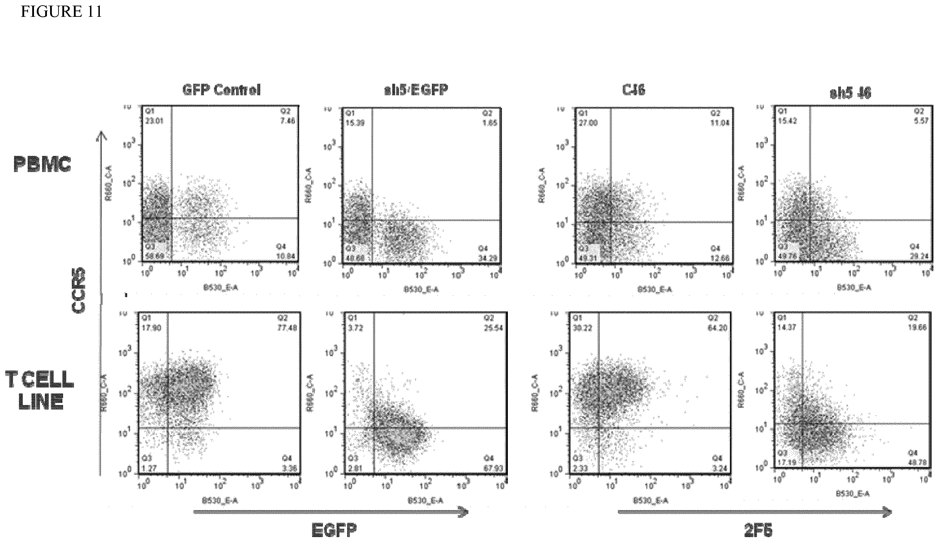

**Figure 11****. Comparison of gene expression in transduced PBMC (at day 4) and transduced CEM.NKR.CCR5 T cells (at week 8).** Similar expression patterns are observed between the two cell types. GFP expression is seen in cells transduced with the constructs containing EGFP (panels 1,2); CCR5 expression is reduced in cells transduced with the constructs containing sh5 (panels 2 and 4), and C46 expression (measured by 2F5 antibody) is observed in cells transduced with the constructs containing C46 (panels 3,4).

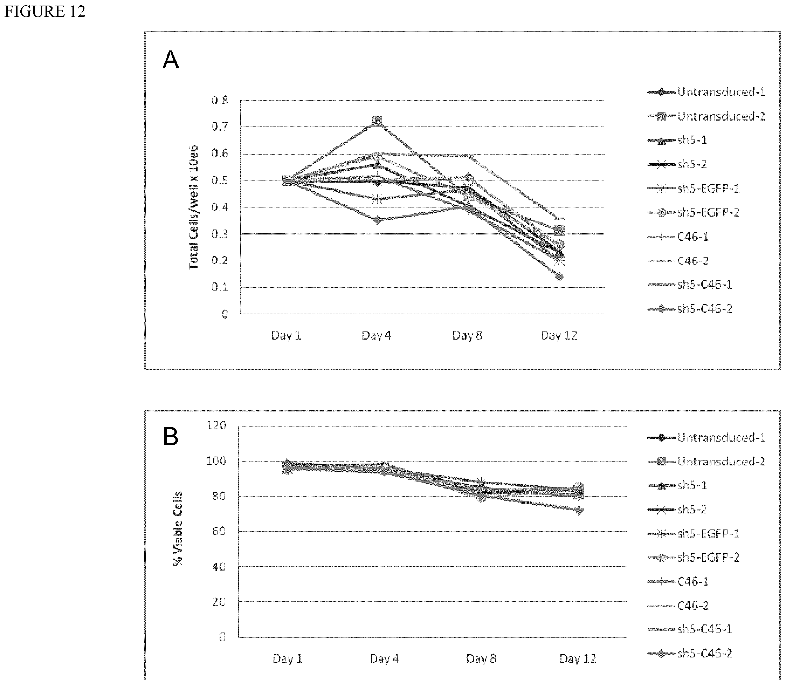

**Figure 12****. Growth Characteristics of Transduced Human PBMC.** Total cells/well (**panel A**) and percentage of viable cells (**panel B**) were similar for PBMC transduced with each of the indicated constructs and PBMC that were not transduced. Two replicate seeds of each group are shown.

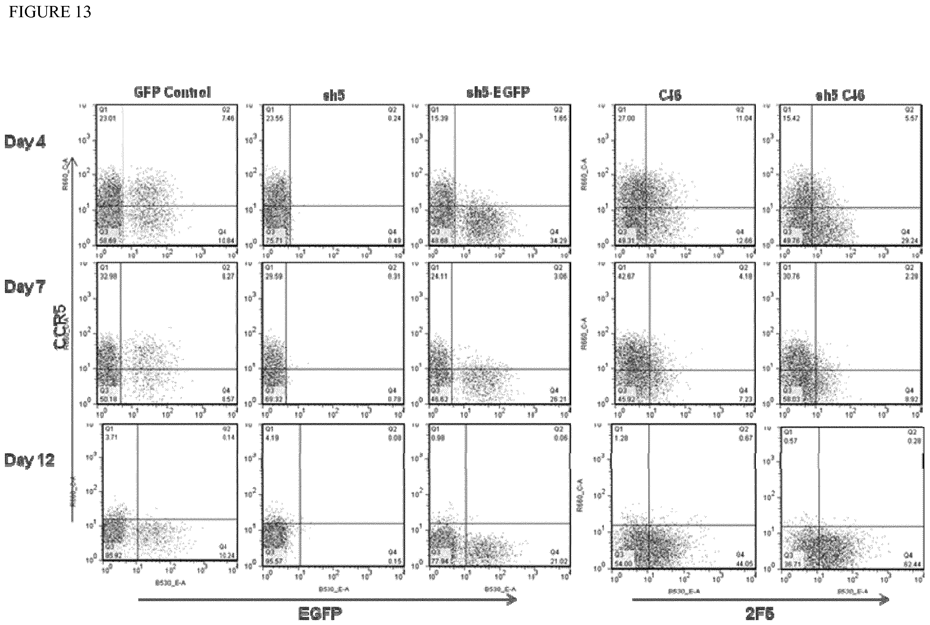

**Figure 13****. Stability of Transgene Expression in PBMC.** FACS analysis of PBMC transduced with the indicated constructs at 4, 7 and 12 days post transduction. GFP, CCR5, and C46 expression (as measured by 2F5 antibody) were analyzed. GFP expression is seen in panels 1,3; sh5 expression is seen in panels 2,3,5 and C46 expression is seen in panels 4,5.

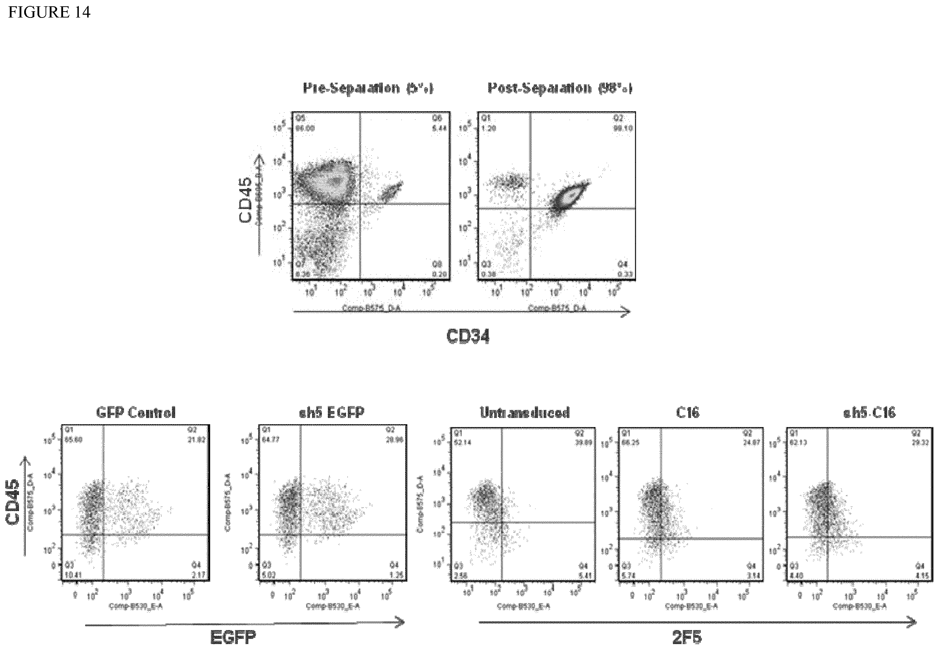

**Figure 14****. CD34+ Isolation and Transduction.** FACS analysis of human mononuclear cell populations before (pre-scparation) and after (post-separation) isolation of CD34+ cells by Magnetic Antibody Cell Separation (upper panel). FACS analysis of human CD34+ hematopoietic stem cells transduced with the indicated constructs (bottom panel). GFP expression is seen in panels 1,2; C46 expression is not seen in panels 4,5.

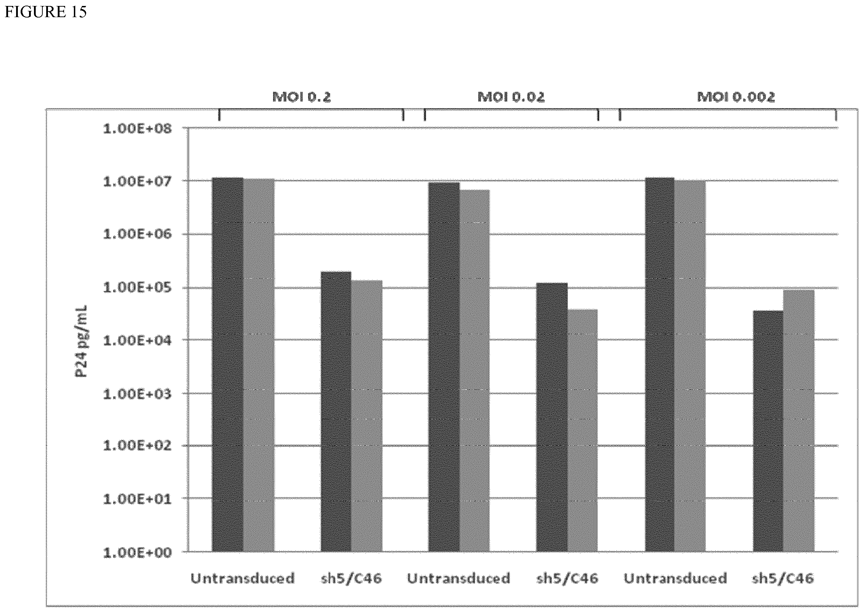

**Figure 15****. HIV Challenge with Dual Tropic SF2 Strain in Molt4/CCR5 Cells.** Molt4/CCR5 cells were either non-transduced or transduced with sh5/C46 lentiviral vector and subsequently challenged with HIV-SF2 dual tropic (CCR5 and CXCR4) virus at varying multiplicity of infection (MOI) - 0.2, 0.02, 0.002. P24 protein levels were assessed 13 days after viral challenge as a measure of HIV infection.

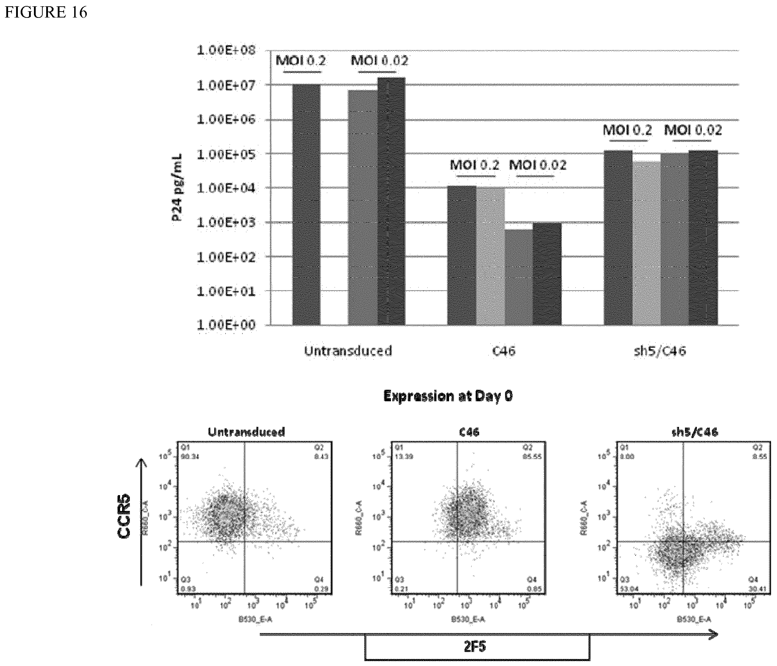

**Figure 16****. HIV Challenge with Dual Tropic SF2 Strain in Molt4/CCR5 Cells.** Molt4/CCR5 cells were either non-transduced or transduced with sh5/C46 or C46 lentiviral constructs and subsequently challenged with HIV-SF2 dual tropic (CCR5 and CXCR4) virus at two different multiplicity of infection (MOI) 0.2 and 0.02. P24 protein levels were assessed 11 days after viral challenge as a measure of HIV infection (upper panel). FACS analysis of non-transduced Molt4/CCR5 cells or Molt4/CCR5 cells transduced with C46 or sh5/C46 lentiviral constructs on the day of viral challenge (lower panel). CCR5 and C46 (as measured by 2F5 antibody) expression was assessed.

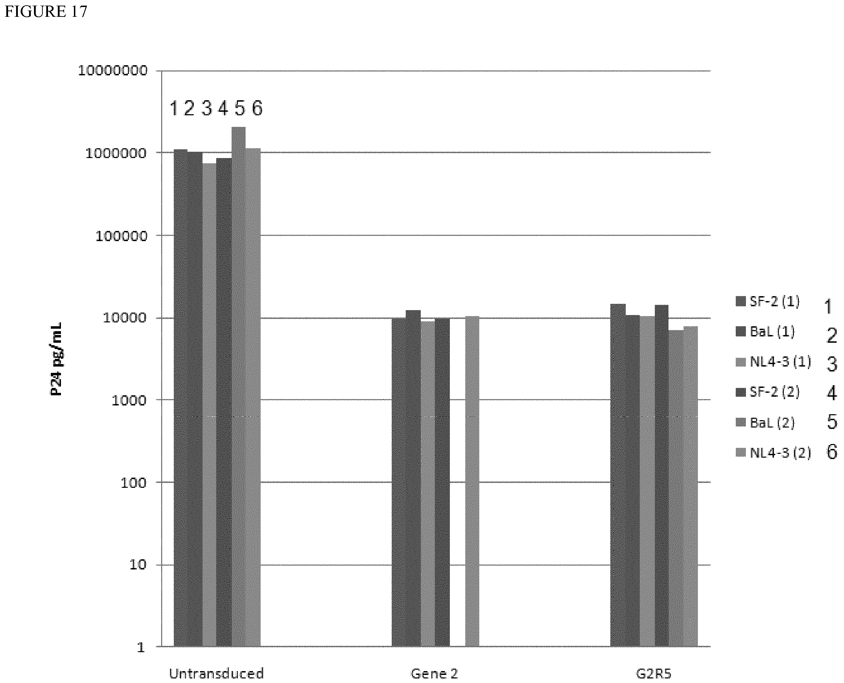

**Figure 17****. HIV Challenge with Dual Tropic SF2 Strain in Molt4/CCR5 Cells.** Molt4/CCR5 cells were either non-transduced or transduced with C46 (Gene 2) or sh5/C46 (G2R5) lentiviral constructs and subsequently challenged with HIV-SF2 dual tropic (CCR5 and CXCR4), Bal (CCR5 tropic) or NL4-3 (CXCR4 tropic) virus at an MOI of 0.2. P24 protein levels were assessed 11 days after viral challenge as a measure of HIV infection. The numbering on the histograms (1-6) refers to the HIV strains that were used (see key on right-hand side).

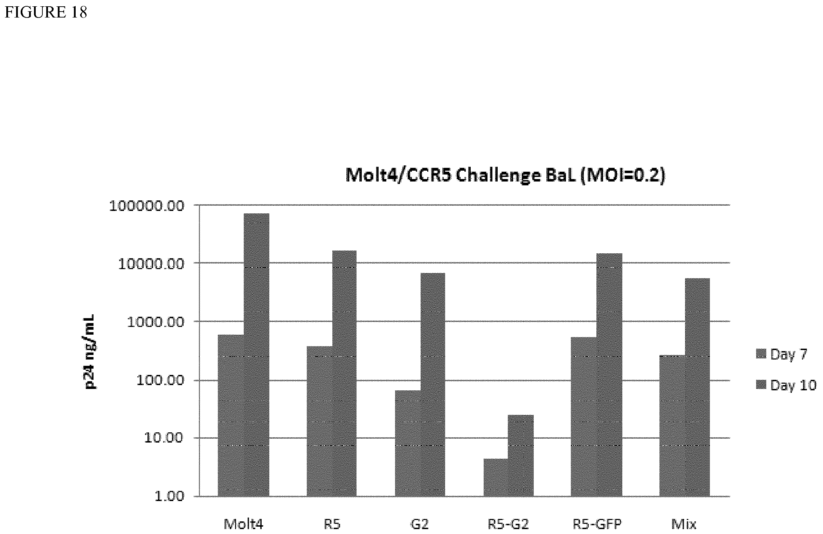

**Figure 18****. HIV Challenge with CCR5 Tropic Bal Strain in Molt4/CCR5 Cells.** Molt4/CCR5 cells were either non-transduced (Molt4) or transduced with one of the following four lentiviral constructs: sh5 (R5); C46 (G2); sh5/C46 (R5-G2); or sh5/EGFP (R5-GFP). The "mix" group is a mixture of untransduced, sb5, C46, sh5/C46 all mixed equally (i.e. 25% of each type). The cells were subsequently challenged with HIV-Bal CCR5 tropic virus at a multiplicity of infection (MOI) of 0.2. P24 protein levels were assessed 7 and 10 days (first and second histogram respectively for each treatment) after viral challenge as a measure of HIV infection.

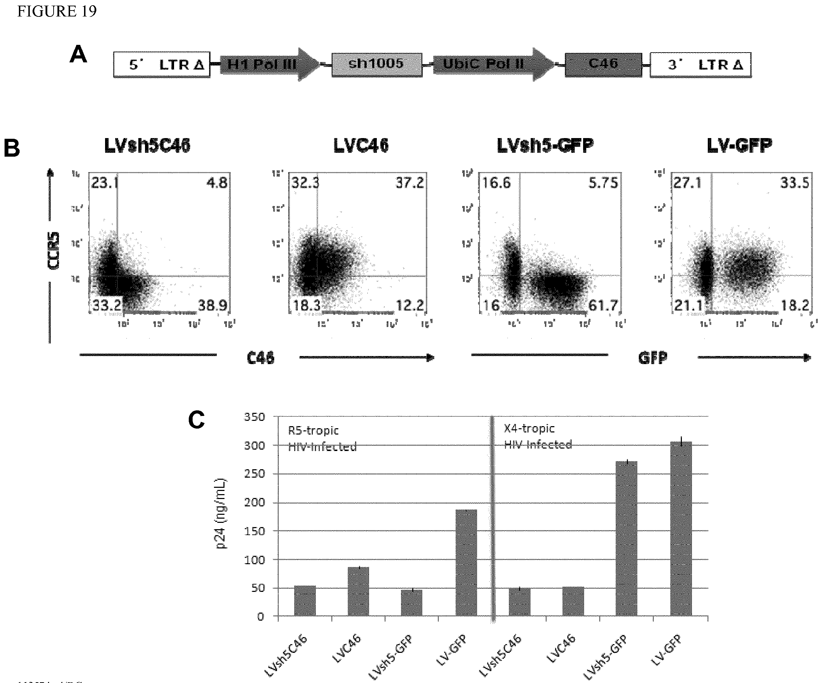

**Figure 19****. HIV Challenge in Peripheral Blood Mononuclear Cells. A**. Diagram of Dual sh1005/C46 Construct. **B.** PBMC were transduced with one of the following four lentiviral constructs: sh5/C46 (LVsh5C46); C46 (LVC46); sh5/GFP (LVsh5-GFP); or GFP control (LV-GFP). FACS analysis of transduced PBMC four days post transduction. C. Sixteen days post transduction, cells shown in panel B were challenged with either a CCR5 (R5)-tropic or CXCR4 (X4)-tropic HIV strain and p24 protein levels were assessed in culture supernatants four days following viral challenge.

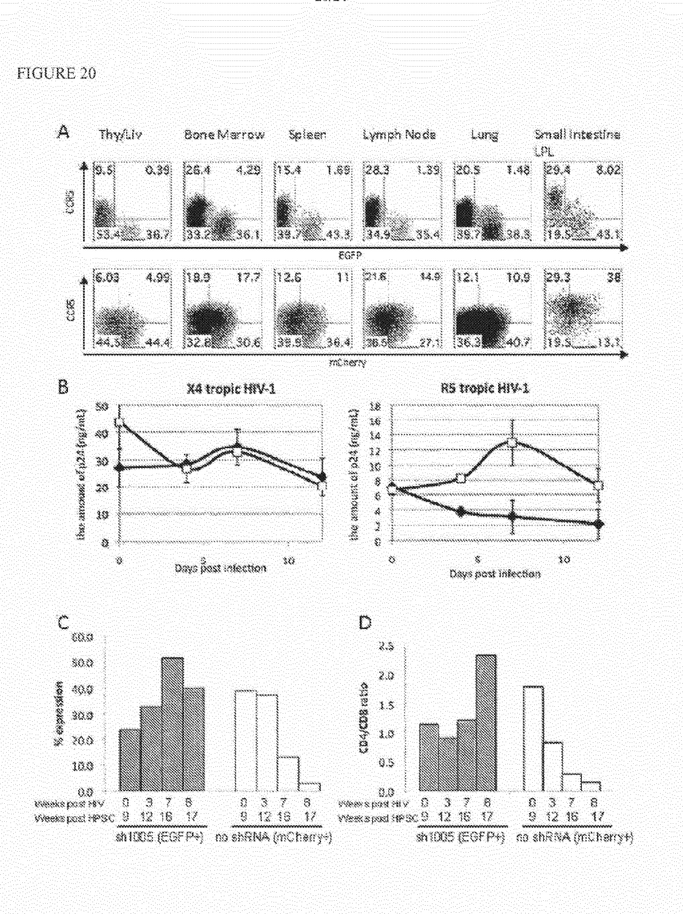

**Figure 20****. Efficient CCR5 reduction in the NOD SCID-hu BLT mouse. A. Flow Cytometry.** The percent CCR5 expression in EGFP+ (upper panel) and mCherry+ (lower panel) CD4+ T-cells was examined by FACS analysis in lymphoid organs of reconstituted mice. Representative data from a mouse at 20 weeks post reconstitution is shown. Thy/Liv: Transplanted human thymus like organoid. LPL: Lamina propria lymphocytes. **B. CCR5 tropic HIV-1 inhibition *ex vivo.*** Splenocytes isolated from a transplanted mouse were activated with PHA for 2 days and IL-2 for 5 days and CD8+ cells were depleted. Cells were sorted for EGFP+ and mCherry+ at over 99% purities. Sorted EGFP+ (black diamond) and mCherry+ (open square) cells (4 x 10⁴) were infected with R5 HIV-1_{NFNSXSL9} or X4 HIV-1_{NL4-3} at MOI of 2.5 in parallel and in triplicate. Cells were washed 3 times after the infection. The amount of remaining input HIV-1 particles in culture supernatant was monitored 1 hour after infection by HIV p24 ELISA assay. The amount of HIV production in culture supernatant was monitored at day 4, 7 and 12 after infection during the culture. **C. Selective advantage of CCR5 downregulated CD4+ T-cells *in vivo.*** Reconstituted mice were infected with R5 tropic HIV-1_{NFNSXSL9} (dose=200 ng of p24) at 9 weeks post HPSC transplant. Kinetics of % EGFP+ CD4+ T-cell population (Gray bar) in peripheral blood was monitored for 8 weeks after R5 tropic HIV injection. % mCherry+ CD4+ T-cell population (White bar) was monitored within the same animal. The % EGFP+ and % mCherry+ were maintained in HIV uninfected mice at 17 week post HPSC transplant (data not shown). Representative data is shown. **D. Selective maintenance of CD4/CD8 ratio *in vivo.*** Kinetics of CD4/CD8 ratio in EGFP+ CD45+ T-cell population (Gray bar) in peripheral blood was monitored during 8 weeks after R5 tropic HIV injection. CD4/CD8 ratio in % mCherry+ CD4+ T-cell population (White bar) was monitored within the same animal. A representative animal is shown. The CD4/CD8 ratio in EGFP+ and mCherry+ CD45+ cells were maintained above 1 in HIV uninfected mice at 17 week post HPSC transplant (data not shown).

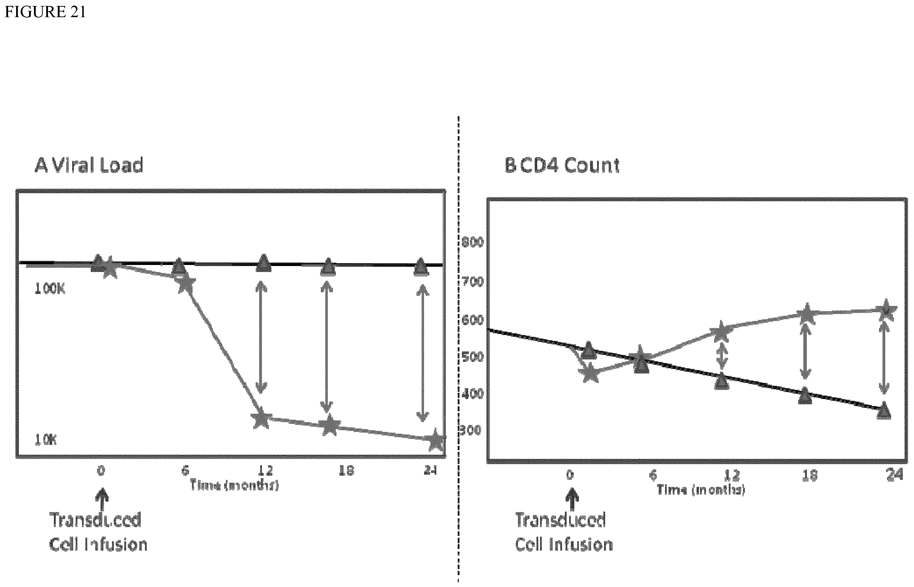

**Figure 21****. Predicted impact of introducing sh5/C46-transduced CD34+ and/or CD4+ cells into an HIV+ individual naive to HAART.** Predicted viral load (**A**) and CD4 count (**B**) in patients treated with one dose of transduced cells (stars) versus untreated patients (triangles).

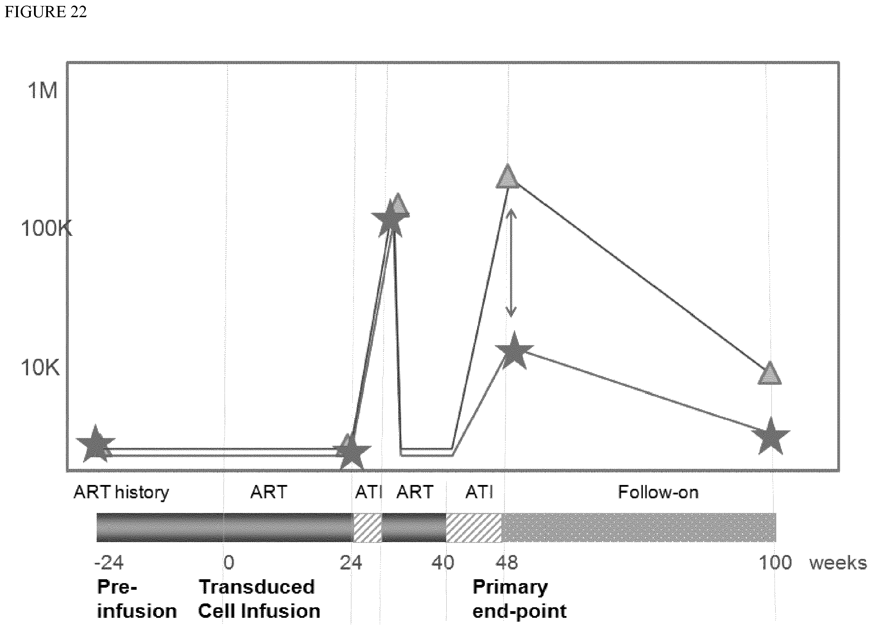

**Figure 22****. Predicted impact of introducing sh5/C46-transduced CD34+ and/or CD4+ cells into an HIV+ individual on a well-controlled HAART regimen.** The y-axis depicts predicted viral load. The x-axis details when antiretroviral therapy (ART) is being taken or when an analytic treatment interruption (ATI) is instituted. Predicted viral loads for patients treated with one dose of transduced cells (stars) versus untreated patients (triangles) is shown.

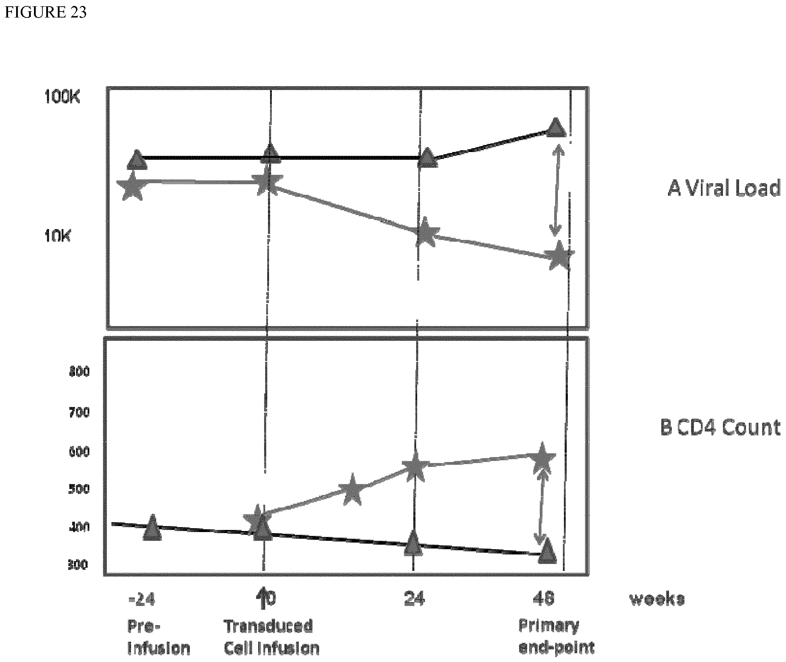

**Figure 23****. Predicted impact of introducing sh5/C46-transduced CD34+ and/or CD4+ cells into an HIV+ individual failing HAART.** Predicted viral load (**A**) and CD4 count (**B**) in patients treated with one dose of transduced cells (stars) versus untreated patients (triangles).

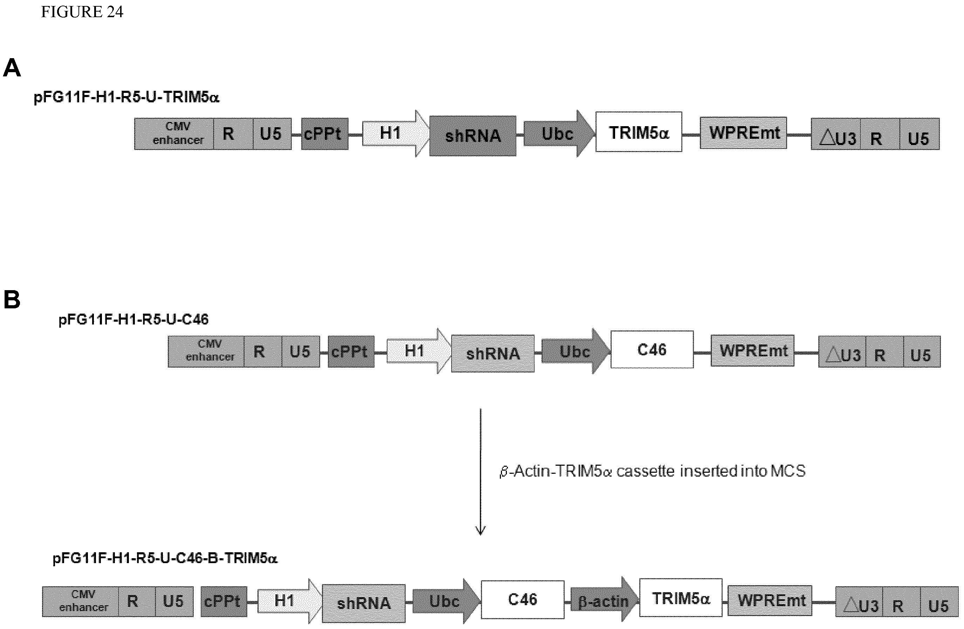

**Figure 24****. Dual Lentiviral Construct with shRNA targeting CCR5 and a TRIM5α protein. A.** Schematic showing the elements of pFG11F-H1-R5-U-TRIM5α. **B.** Schematic showing the derivation of the triple vector pFG11F-H1-R5-U-C46-B-TRIM5α.

### DETAILED DESCRIPTION OF THE INVENTION

The present invention is based, in part, on the recognition that a therapeutic approach that targets non-HIV genes and/or proteins (that is host cell genes and/or proteins) decreases the probability that new HIV strains resistant to the inhibitors will emerge. In particular, the present invention provides vectors and methods of using such vectors to prevent or treat HIV infection by targeting or employing cellular proteins that affect different stages of the HIV life cycle. For instance, in one embodiment, the vector is capable of expressing an inhibitor of viral entry (binding) and an inhibitor of viral fusion to the cell membrane. In another embodiment, the vector is capable of expressing an inhibitor of viral entry and an inhibitor of viral replication. Accordingly, the present invention provides an expression vector comprising a first nucleic acid sequence encoding an inhibitor of an HIV co-receptor and a second nucleic acid sequence encoding a protein that inhibits HIV viral fusion to a target cell or HIV replication.

In one particular embodiment, all three elements (*e.g*., an inhibitor of an HIV co-receptor, a protein that inhibits HIV viral fusion to a target cell and a protein that inhibits HIV replication) are combined in one vector. For instance, in one embodiment, the expression vector comprises a first, second, and third nucleic acid sequence, wherein the first nucleic acid sequence encodes an inhibitor of an HIV co-receptor (*e.g*., shRNA to CCR5 or CXCR4), the second nucleic acid sequence encodes a fusion inhibitor (*e.g*., C46), and the third nucleic acid sequence encodes an inhibitor of HIV replication (*e.g.,* TRIM5α protein or a derivative or fusion thereof). In another embodiment, the expression vector comprises a first, second, and third nucleic acid sequence, wherein the first nucleic acid sequence encodes a first inhibitor of an HIV co-receptor (*e.g*., shRNA to CCR5), the second nucleic acid sequence encodes a second inhibitor of an HIV co-receptor (e*.g.*, shRNA to CXCR4), and the third nucleic acid sequence encodes an inhibitor of HIV viral fusion to a target cell (*e.g*., C46).

As used herein, "expression vector" or "vector" refers to a composition of matter which can be used to deliver nucleic acids of interest to the interior of a cell such that they will be expressed by the cell. Numerous vectors are known in the art including, but not limited to, linear polynucleotides, polynucleotides associated with ionic or amphiphilic compounds, plasmids, and viral vectors. Examples of viral vectors include, but are not limited to, adenoviral vectors, adeno-associated virus vectors, retroviral vectors (including lentiviral vectors), and the like. In one embodiment, the expression vector is a viral vector. Preferably, the viral vector is a retroviral or lentiviral vector.

"Retroviruses" are viruses having an RNA genome that is reverse transcribed by retroviral reverse transcriptase to a cDNA copy that is integrated into the host cell genome. Retroviral vectors and methods of making retroviral vectors are known in the art. Briefly, to construct a retroviral vector, a nucleic acid encoding a gene of interest is inserted into the viral genome in the place of certain viral sequences to produce a virus that is replication-defective. In order to produce virions, a packaging cell line containing the gag, pol, and env genes but without the LTR and packaging components is constructed (Mann et al., Cell, Vol. 33:153-159, 1983). When a recombinant plasmid containing a cDNA, together with the retroviral LTR and packaging sequences, is introduced into this cell line, the packaging sequence allows the RNA transcript of the recombinant plasmid to be packaged into viral particles, which are then secreted into the culture media. The media containing the recombinant retroviruses is then collected, optionally concentrated, and used for gene transfer (see Example 1).

"Lentivirus" refers to a genus of retroviruses that is capable of infecting dividing and non-dividing cells. Several examples of lentiviruses include HIV (human immunodeficiency virus: including HIV type 1, and HIV type 2), the etiologic agent of the human acquired immunodeficiency syndrome (AIDS); visna-maedi, which causes encephalitis (visna) or pneumonia (maedi) in sheep, the caprine arthritis-encephalitis virus, which causes immune deficiency, arthritis, and encephalopathy in goats; equine infectious anemia virus, which causes autoimmune hemolytic anemia, and encephalopathy in horses; feline immunodeficiency virus (FIV), which causes immune deficiency in cats; bovine immune deficiency virus (BIV), which causes lymphadenopathy, lymphocytosis, and possibly central nervous system infection in cattle; and simian immunodeficiency virus (SIV), which causes immune deficiency and encephalopathy in sub-human primates.

A "hybrid virus" as used herein refers to a virus having components from one or more other viral vectors, including elements from non-retroviral vectors, for example, adenoviral-retroviral hybrids. As used herein hybrid vectors having a retroviral component are to be considered within the scope of the retroviruses.

A "pseudotyped" retrovirus is a retroviral particle having an envelope protein that is from a virus other than the virus from which the RNA genome is derived. The envelope protein may be from a different retrovirus or from a non-retroviral virus. A preferred envelope protein is the vesicular stomatitis virus G (VSV G) protein. However, to eliminate the possibility of human infection, viruses can alternatively be pseudotyped with ecotropic envelope protein that limit infection to a specific species, such as mice or birds. For example, in one embodiment, a mutant ecotropic envelope protein is used, such as the ecotropic envelope protein 4.17 (Powell et al. Nature Biotechnology 18(12):1279-1282 (2000)).

The term "provirus" is used to refer to a duplex DNA sequence present in a eukaryotic chromosome that corresponds to the genome of an RNA retrovirus. The provirus may be transmitted from one cell generation to the next without causing lysis or destruction of the host cell.

A lentiviral genome is generally organized into a 5' long terminal repeat (LTR), the gag gene, the pol gene, the env gene, the accessory genes (nef, vif, vpr, vpu) and a 3' LTR. The viral LTR is divided into three regions called U3, R and US. The U3 region contains the enhancer and promoter elements. The U5 region contains the polyadenylation signals. The R (repeat) region separates the U3 and U5 regions and transcribed sequences of the R region appear at both the 5' and 3' ends of the viral RNA. See, for example, "RNA Viruses: A Practical Approach" (Alan J. Cann, Ed. , Oxford University Press, (2000)); O Narayan and Clements (1989) J. Gen. Virology, Vol. 70:1617-1639; Fields et al. (1990) Fundamental Virology Raven Press.; Miyoshi H, Blamer U, Takahashi M, Gage F H, Verma I M. (1998) J Virol., Vol. 72(10):8150 7, and U.S. Pat. No. 6,013,516.

Lentiviral vectors are known in the art, including several that have been used to infect hematopoietic progenitor/stem, cells (HPSC). Such vectors can be found, for example, in the following publications, which are incorporated herein by reference: Evans et al., Hum Gene Ther., Vol. 10:1479-1489, 1999; Case et al., Proc Natl Acad Sci USA, Vol. 96:2988-2993, 1999; Uchida et al., Proc Natl Acad Sci USA, Vol. 95:11939-11944, 1998; Miyoshi et al., Science, Vol. 283:682-686, 1999; and Sutton et al., J. Virol., Vol. 72:5781-5788, 1998. In one embodiment, the expression vector is a modified lentivirus, and thus is able to infect both dividing and non-dividing cells. Such lentiviral vectors comprise a modified lentiviral genome that comprises a first nucleic acid sequence encoding an inhibitor of an HIV co-receptor and a second nucleic acid sequence encoding a protein that inhibits HIV fusion to a target cell or HIV replication. Further, the modified lentiviral genome preferably lacks genes for lentiviral proteins required for viral replication, thus preventing undesired replication, such as replication in the target cells. The required proteins for replication of the modified genome are preferably provided in trans in the packaging cell line during production of the recombinant retrovirus (or specifically lentivirus). In one embodiment, the packaging cell line is a 293T cell line. The lentiviral vector preferably comprises sequences from the 5' and 3' long terminal repeats (LTRs) of a lentivirus. In one embodiment, the viral construct comprises the R and U5 sequences from the 5' LTR of a lentivirus and an inactivated or self-inactivating 3' LTR from a lentivirus. The LTR sequences may be LTR sequences from any lentivirus including from any species or strain. For example, the LTR may be LTR. sequences from HIV, simian immunodeficiency virus (SIV), feline immunodeficiency virus (FIV) or bovine immunodeficiency virus (BIV). Preferably the LTR sequences are HIV LTR sequences.

In certain embodiments, the lentiviral vector comprises an inactivated or self-inactivating 3' LTR-that is the lentiviral vector is self-inactivating. A "self-inactivating 3' LTR" is a 3' LTR that contains a mutation, substitution or deletion that prevents the LTR sequences from driving expression of a downstream gene. A copy of the U3 region from the 3' LTR acts as a template for the generation of both LTRs in the integrated provirus. Thus, when the 3' LTR with an inactivating deletion or mutation integrates as the 5' LTR of the provirus, no transcription from the 5' LTR is possible. This eliminates competition between the viral enhancer/promoter and any internal enhancer/promoter. Self-inactivating 3' LTRs are described, for example, in Zufferey et al., J. Virol., Vol. 72:9873-9880,1998; Miyoshi et al., J. Virol., Vol. 72:8150-8157, 1998; and Iwakuma et al., Virology, Vol. 261:120-132, 1999. The 3' LTR may be made self-inactivating by any method known in the art. In one embodiment the U3 element of the 3' LTR contains a deletion of its enhancer sequence, preferably the TATA box, Spl and NF-kappa B sites. As a result of the self-inactivating 3' LTR, the provirus that is integrated into the host cell genome will comprise an inactivated 5' LTR. The viral expression vectors of the invention preferably do not inhibit vector production in producer cells. In certain embodiments, the viral expression vector substantially lacks toxicity to transduced and gene-containing cells.

The expression vector of the invention comprises a first nucleic acid sequence encoding an inhibitor of an HIV co-receptor. In one embodiment, the HIV co-receptor is CC chemokine receptor 5 (CCR5). CCR5 is the primary HIV-1 co-receptor for macrophage tropic strains and is essential for HIV infection. Population genetic studies have demonstrated that individuals homozygous for a defective CCR5 gene (*e.g*. CCR5A32) are protected from HIV infection. Interestingly, heterozygous individuals who exhibit a 50% reduction of CCR5 on cells have a substantially reduced disease progression rate. Individuals who are homozygous for the CCR5Δ32 allele appear to be normal except for an increased susceptibility to West Nile virus encephalitis. A small molecule CCR5 inhibitor, Maraviroc has been approved by the FDA for use in humans. This inhibitor is effective in preventing HIV-1 infection and although some adverse effects were noted, there did not appear to be any such effects resulting from blocking CCR5 itself. As expected, HIV-1 resistance does occur, however, interestingly, the major form of resistance appears to be HIV-1 variants that adapt to use the drug-occupied form of CCR5 rather than CXC chemokine receptor 4 (CXCR4) or other co-receptors. Thus, knockdown of CCR5 (such as with siRNA, shRNA, or antisense) may be more effective than blocking access. In another embodiment of the invention, the HIV co-receptor targeted is CXCR4, which is the major co-receptor for T-cell tropic strains.

In certain embodiments, the inhibitor of an HIV co-receptor is an inhibitory nucleic acid. As used herein, an inhibitory nucleic acid includes, but is not limited to, a small interfering RNA (siRNA), a short hairpin RNA (shRNA), an aptamer, a ribozyme, and an antisense oligonucleotide. Thus, in one embodiment, the first nucleic acid sequence encodes an inhibitory nucleic acid that targets an HIV co-receptor. "Target" refers to the ability of the inhibitor to bind to and/or interfere with an endogenous transcript encoding the HIV co-receptor, For instance, the inhibitory nucleic acid can have a sequence that is substantially complementary to a nucleic acid encoding the HIV co-receptor such that the inhibitory nucleic acid binds to the HIV co-receptor-encoding nucleic acid thereby blocking the expression or initiating the degradation of the co-receptor nucleic acid. Accordingly, in some embodiments, the inhibitor of an HIV co-receptor is capable of reducing expression of the HIV co-receptor when the expression vector encoding said inhibitor is expressed in a host cell.

A "small interfering RNA" or "siRNA" is a double-stranded RNA molecule that is capable of inhibiting the expression of a gene with which it shares homology. The region of the gene or other nucleotide sequence over which there is homology is known as the "target region." In one embodiment the siRNA may be a "hairpin" or stem-loop RNA molecule (shRNA), comprising a sense region, a loop region and an antisense region complementary to the sense region. In other embodiments the siRNA comprises two distinct RNA molecules that are non-covalently associated to form a duplex.

In some embodiments, an expression vector of the invention comprises a first nucleic acid sequence encoding an antisense oligonucleotide having a sequence that is substantially complementary to at least a portion of a nucleic acid sequence encoding an HIV co-receptor, such as CCR5 and/or CXCR4. As used herein, "substantially complementary" refers to a sequence that is at least about 75%, 80%, 85%, 90%, 95%, 96%, 97%, 98%, or 99% complementary to a target polynucleotide sequence. In one embodiment, the antisense oligonucleotide has a sequence that is 100% complementary to at least a portion of a nucleic acid sequence encoding CCR5 or CXCR4. The antisense oligonucleotide can be from about 15 to about 30 nucleotides in length, and in some embodiments about 19 to about 25 nucleotides in length.

In other embodiments, an expression vector of the invention comprises a first nucleic acid sequence encoding a siRNA or shRNA. The siRNA or shRNA preferably has a double-stranded region comprising a sequence that is substantially identical and complementary to a portion of a nucleic acid sequence encoding an HIV co-receptor, that is at least about 75%, 80%, 85%, 90%, 95%, 96%, 97%, 98%, or 99% identical and complementary to a portion of a nucleic acid encoding CCR5 or CXCR4. In one embodiment, the siRNA or shRNA has a double-stranded region comprising a sequence that is 100% identical and complementary to a HIV co-receptor sequence (*e.g*. CCR5 and/or CXCR4). The double-stranded region of the siRNA or shRNA can be from about 5 to about 60 nucleotides in length, preferably about 10 to about 30 nucleotides in length, more preferably about 15 to about 25 nucleotides in length, such as about 20 nucleotides in length. In certain embodiments, the first nucleic acid sequence of the expression vector encodes a shRNA having a stem-loop structure, wherein the stem or double-stranded region is substantial identical and complementary to a sequence of CCR5 or CXCR4. The loop region of the shRNA can comprise from about 2 to about 15 nucleotides. In one particular embodiment, the first nucleic acid sequence encodes a shRNA comprising a sequence of 5'-AGCAAGCUC AGUUUACACC UUGUCCGACG GUGUAAACUG AGCUUGCUCU U-3' (SEQ ID NO:1).

The expression vector of the invention preferably comprises a second nucleic acid sequence encoding a protein that inhibits HIV fusion to a target cell or HIV replication. In some embodiments, the protein that inhibits HIV fusion to a target cell is a C46 protein. C46 is a membrane anchored fusion inhibitor derived from the C-terminal heptad repeat of HIV gp41 fused with a human immunoglobulin hinge region and a CD34 transmembrane domain. C46 is a potent HIV fusion inhibitor, in a sense analogous to the FDA approved soluble drug enfuvirtide (T20) and acts at a point in the HIV life cycle distinct from CCR5 co-receptor attachment. In one embodiment, the safety of C46 was tested in a phase I clinical trial in which patients received an infusion of autologous T-cells transduced with C46 retroviral vector. In another aspect, the patients had no gene therapy related adverse effects and did not develop apparent anti-C46 immune reactions. In one embodiment, the second nucleic acid sequence encodes a C46 protein comprising a sequence of: 


 (SEQ ID NO: 2).
 In another embodiment, the second nucleic acid comprises a sequence of: 
5'-ATGGGAGCAG GAGCAACCGG AAGGGCAATG GACGGACCAC GGCTGCTGCT GCTGCTGCTG
CTGGGCGTGA GCCTGGGCGG CGCCCGGAGC TGGATGGAGT GGGACCGGGA GATCAACAAC
TACACCAGCC TGATCCACAG CCTGATCGAG GAGAGCCAGA ACCAGCAGGA GAAGAACGAG
CAGGAGCTGC TGGAGCTGGA CAAGTGGGCC AGCCTGTGGA ACTGGTTCCG GAGCGAGCGG
AAGTGCTGCG TGGAGTGCCC ACCATGCCCA GCACCACCAG TGGCAGGACC CCTGATCGCA
 
CTGGTGACCA GCGGAGCCCT GCTGGCCGTG CTGGGCATCA CAGGCTACTT CCTGATGAAC
CGGCGGAGCT GGAGCCCCAC CGGCGAGCGG CTGGAGCTGG AGCCCTGA-3' (SEQ ID NO: 3).
 Other suitable proteins that inhibit HIV fusion to a target cell and can be encoded by the second nucleic acid sequence in the expression vectors of the invention include T20 and its related proteins, enfuvirtide, CP₃₂M, and sifuvirtide.

In certain embodiments, the second nucleic acid sequence encodes a protein that inhibits HIV replication. In some embodiments, the protein that inhibits HIV replication is a tripartite motif-containing 5 alpha (TRIM5α) protein or derivatives or fusions thereof. For instance, the second nucleic acid sequence can encode a human TRIM5α, rhesus TRIM5α, a chimeric TRIM5α, or a human TRIM5-cyclophilin fusion protein. In one embodiment, the second nucleic acid sequence encodes a human TRIM5α protein comprising a sequence of: 
MASGILVNVK EEVTCPICLE LLTQPLSLDC GHSFCQACLT ANHKKSMLDK GESSCPVCRI

SYQPENIRPN RHVANIVEKL REVKLSPEGQ KVDHCARHGE KLLLFCQEDG KVICWLCERS

QEHRGHHTFL TEEVAREYQV KLQAALEMLR QKQQEAEELE ADIREEKASW KTQIQYDKTN

VLADFEQLRD ILDWEESNEL QNLEKEEEDI LKSLTNSETE MVQQTQSLRE LISDLEERLQ

GSVMELLQGV DGVIKRTENV TLKKPETFPK NQRRVFRAPD LKGMLEVFRE LTDVRRYWVD

VTVAPNNISC AVISEDKRQV SSPKPQIIYG ARGTRYQTFV NFNYCTGILG SQSITSGKHY

WEVDVSKKTA WILGVCAGFQ PDAMCNIEKN ENYQPKYGYW VIGLEEGVKC SAFQDSSFHT

PSVPFIVPLS VIICPDRVGV FLDYEACTVS FFNITNHGFL IYKFSHCSFS QPVFPYLNPR

KCGVPMTLCS PSS (SEQ ID NO: 4).
 In another embodiment, the second nucleic acid sequence comprises a sequence of:

5'-ATGGCTTCTG GAATCCTGGT TAATGTAAAG GAGGAGGTGA CCTGCCCCAT CTGCCTGGAA
CTCCTGACAC AACCCCTGAG CCTGGACTGC GGCCACAGCT TCTGCCAAGC ATGCCTCACT
GCAAACCACA AGAAGTCCAT GCTAGACAAA GGAGAGAGTA GCTGCCCTGT GTGCCGGATC
AGTTACCAGC CTGAGAACAT ACGGCCTAAT CGGCATGTAG CCAACATAGT GGAGAAGCTC
AGGGAGGTCA AGTTGAGCCC AGAGGGGCAG AAAGTTGATC ATTGTGCACG CCATGGAGAG
AAACTTCTAC TCTTCTGTCA GGAGGACGGG AAGGTCATTT GCTGGCTTTG TGAGCGGTCT
CAGGAGCACC GTGGTCACCA CACGTTCCTC ACAGAGGAGG TTGCCCGGGA GTACCAAGTG
AAGCTCCAGG CAGCTCTGGA GATGCTGAGG CAGAAGCAGC AGGAAGCTGA AGAGTTAGAA
GCTGACATCA GAGAAGAGAA AGCTTCCTGG AAGACTCAAA TACAGTATGA CAAAACCAAC
GTCTTGGCAG ATTTTGAGCA ACTGAGAGAC ATCCTGGACT GGGAGGAGAG CAATGAGCTG
CAAAACCTGG AGAAGGAGGA GGAAGACATT CTGAAAAGCC TTACGAACTC TGAAACTGAG

ATGGTGCAGC AGACCCAGTC CCTGAGAGAG CTCATCTCAG ATCTGGAGCA TCGGCTGCAG
GGGTCAGTGA TGGAGCTGCT TCAGGCTGTG GATGGCGTCA TAAAAAGGAC GGAGAACGTG
ACCTTGAAGA AGCCAGAAAC TTTTCCAAAA AATCAAAGGA GAGTGTTTCG AGCTCCTGAT
CTGAAAGGAA TGCTAGAAGT GTTTAGAGAG CTGACAGATG TCCGACGCTA CTGGGTTGAT
GTGACAGTGG CTCCAAACAA CATTTCATGT GCTGTCATTT CTGAAGATAA GAGACAAGTG
AGCTCTCCGA AACCACAGAT AATATATGGG GCACGAGGGA CAAGATACCA GACATTTGTG
AATTTCAATT ATTGTACTGG CATCCTGGGC TCTCAAAGTA TCACATCAGG GAAACATTAC
TGGGAGGTAG ACGTGTCCAA GAAAACTGCT TGGATCCTGG GGGTATGTGC TGGCTTCCAA
CCTGATGCAA TGTGTAATAT TGAAAAAAAT GAAAATTATC AACCTAAATA CGGCTACTGG
GTTATAGGGT TAGAGGAAGG AGTTAAATGT AGTGCTTTCC AGGATAGTTC CTTCCATACT
CCTTCTGTTC CTTTCATTGT GCCCCTCTCT GTGATTATTT GTCCTGATCG TGTTGGAGTT
TTCCTAGACT ATGAGGCTTG CACTGTCTCA TTCTTCAATA TCACAAACCA TGGATTTCTC
ATCTATAAGT TTTCTCACTG TTCTTTTTCT CAGCCTGTAT TTCCATATTT AAATCCTAGA
AAATGTGGAG TCCCCATGAC TCTGTGCTCA CCAAGCTCTT GA-3' (SEQ ID NO: 5).

A "chimeric TRIM5α" refers to a TRIM5α protein comprising domains or fragments from TRIM5α proteins from two or more species. For example, a chimeric TRIM5α can comprise at least one domain from a human TRIM5α and at least one domain from a rhesus TRIM5α. In some embodiments, a chimeric TRIM5α protein comprises an amino terminal domain from a human TRIM5α protein and a carboxy terminal PRYSPRY domain from a rhesus TRIM5α protein.

In another embodiment, the second nucleic acid sequence encodes a fusion protein comprising TRIM 5α and cyclophilin. For instance, in one embodiment, the TRIM5-cyclophilin fusion protein comprises amino acids 1 to about 309 of human TRIM5α fused directly to about full-length human cyclophilin A. In another embodiment, the TRIM5-cyclophilin fusion protein comprises amino acids 1 to about 322 of human TRIM5α fused directly to about full-length human cyclophilin A. In still another embodiment, the TRIM5-cyclophilin fusion protein comprises amino acids 1 to about 33 1 of human TRIM5α fused directly to about full-length human cyclophilin A. Other suitable proteins that inhibit HIV replication that can be encoded by the second nucleic acid sequence include, but are not limited to, cyclophilin, E3 ubiquitin, APOBEC3G, and bone marrow stromal cell antigen 2 (BST-2).

The nucleic acid sequences of the present invention further include nucleic acid sequences that encode conservative variants or functional equivalents of the proteins herein described. As used herein, a conservative variant refers to alterations in the amino acid sequence that do not adversely affect the biological functions of the protein. A substitution, insertion or deletion is said to adversely affect the protein when the altered sequence prevents or disrupts a biological function associated with the protein. For example, the overall charge, structure or hydrophobic/hydrophilic properties of the protein may be altered without adversely affecting a biological activity. Accordingly, the amino acid sequence can be altered, for example to render the protein more hydrophobic or hydrophilic, without adversely affecting the biological activities of the protein,

Ordinarily, the conservative substitution variants and functional equivalents of the proteins, will have an amino acid sequence identity to the disclosed sequences SEQ ID NOs: 2 and 4 of at least about 55%, at least about 65%, at least about 75%, at least about 80%, at least about 85%, at least about 90%, at least about 95%, or at least about 96% to 99%. Identity or homology with respect to such sequences is defined herein as the percentage of amino acid residues in the candidate sequence that are identical with the known peptides, after aligning the sequences and introducing gaps, if necessary, to achieve the maximum percent homology, and not considering any conservative substitutions as part of the sequence identity. N-terminal, C-terminal or internal extensions, deletions, or insertions into the peptide sequence shall not be construed as affecting homology.

Thus, the nucleic acid sequences of the expression vectors of the present invention can encode conservative variants or functional equivalents of the protein sequences described herein. Contemplated variants further include those containing predetermined mutations by, *e.g.,* homologous recombination, site-directed or PCR mutagenesis, and the corresponding proteins of other animal species, including but not limited to rabbit, rat, porcine, bovine, ovine, equine and non-human primate species.

In some embodiments of the expression vectors of the invention, the first nucleic acid sequence encoding an inhibitor of an HIV co-receptor is operably linked to a first promoter and the second nucleic acid sequence encoding a protein that inhibits HIV fusion to a target cell or HIV replication is operably linked to a second promoter. In certain embodiments of the invention in which the expression vector comprises three nucleic acid sequences, each of the three nucleic acid sequences can be operably linked to a separate promoter. For instance, in one embodiment, the first nucleic acid sequence encoding an inhibitor of an HIV co-receptor is operably linked to a first promoter, the second nucleic acid sequence encoding a protein that inhibits HIV fusion to a target cell is linked to a second promoter, and the third nucleic acid sequence encoding a protein that inhibits HIV replication is operably linked to a third promoter. In other embodiments, two of the three nucleic acid sequences are transcribed from a single promoter *(i.e.* the first and second nucleic acid sequences or the second and third nucleic acid sequences). In still other embodiments, all three nucleic acid sequences are transcribed from a single promoter. All three promoters can be the same or different from one another.

The phrase "operably linked" or "under transcriptional control" as used herein means that the promoter is in the correct location and orientation in relation to a nucleic acid sequence to control the initiation of transcription by RNA polymerase and expression of the nucleic acid. The promoters selected preferably lack promoter exclusion, thereby avoiding one promoter switching off the other promoter(s). The first, second, and third promoters can be RNA polymerase I (pol I), polymerase II (pol II), or polymerase III (pol III) promoters. The promoters may be constitutive promoters or inducible promoters. Inducible promoters are known in the art and can include the tetracycline promoter, metallothionein IIA promoter, heat shock promoter, steroid/thyroid hormone/retinoic acid response elements, the adenovirus late promoter, and the inducible mouse mammary tumor virus LTR. In one embodiment, the promoter contains at least a portion of an HIV LTR (*e.g.,* TAR) and is inducible by HIV infection. In certain embodiments, the first promoter is a RNA pol III promoter. RNA pol III promoters suitable for use in the expression vectors of the invention include, but are not limited, to human U6, mouse U6, and human H1. In one embodiment, the first promoter is a H1 RNA pol III promoter. In other embodiments, the second promoter is a RNA pol II promoter, In one particular embodiment, the second promoter is a UbiquitinC pol II promoter. The second promoter, in some embodiments, can be a tissue-specific promoter. For instance, suitable tissue-specific promoters include macrophage-specific promoters (*e.g.,* MPG-1 and the like) and T-cell promoters (*e.g.,* CD4 and the like). In one embodiment, the third promoter is a RNA pol II promoter. In another embodiment, the third promoter is a UbiquitinC pol II promoter. The third promoter can, in some embodiments, be a tissue specific promoter. The first, second, and third promoters can be a combination of any of the promoters described herein. In certain embodiments, RNA pol III promoters are preferred where the nucleic acid sequence encodes an inhibitory RNA molecule, such as an siRNA or shRNA. In other embodiments, RNA pol II promoters are preferred where the nucleic acid sequence encodes a protein.

In embodiments in which the inhibitor of the HIV co-receptor is a siRNA molecule, more than one promoter may be used to generate the siRNA molecule. For instance, in one embodiment, the expression vector comprises one nucleic acid molecule encoding the sense strand of the siRNA molecule and another nucleic acid molecule encoding the antisense strand of the siRNA molecule such that the siRNA duplex is formed following expression of the two nucleic acids. In such embodiments, the expression vector can comprise a first Pol III promoter operably linked to the first nucleic acid encoding the sense strand and a second Pol III promoter operably linked to the second nucleic acid encoding the antisense strand. In another embodiment, the expression vector comprises a first RNA Pol III promoter operably linked to the first nucleic acid sequence encoding the siRNA molecule targeting the HIV co-receptor, and a second RNA Pol III promoter operably linked to the same first nucleic acid sequence in the opposite direction, such that expression of the first nucleic acid sequence from the first RNA Pol III promoter results in a synthesis of the sense strand of the siRNA molecule and expression of the first nucleic acid sequence from the second RNA Pol III promoter results in synthesis of the antisense strand of the siRNA molecule. Following expression of the first nucleic acid sequence from the two different promoters, the sense and antisense strands hybridize to form the duplex siRNA.

In one embodiment, the first nucleic acid sequence and the second nucleic acid sequence are transcribed from a single promoter. For instance, the first nucleic acid sequence and the second nucleic acid sequence are operably linked to a promoter such that a single transcript is generated. In another embodiment, an internal ribosome entry site (IRES) is located upstream of the second nucleic acid sequence and downstream of the first nucleic acid sequence. In other embodiments in which the expression vector comprises three nucleic acid sequences, two of the three nucleic acid sequences are transcribed from a single promoter (*i.e.* the first and second nucleic acid sequences or the second and third nucleic acid sequences). In still other embodiments, all three nucleic acid sequences are transcribed from a single promoter. One or more IRES elements can be present upstream of the second and/or third nucleic acid sequences. For instance, in one embodiment, the first, second, and third nucleic acid sequences can be operably linked to a single promoter and a first IRES element can be positioned between the first and second nucleic acid sequence and a second IRES element can be positioned between the second and third nucleic acid sequence. IRES elements enable efficient translation of polycistronic messages. Any IRES element known in the art can be used in the expression constructs of the invention.

In certain embodiments, the first and second nucleic acid sequences are expressed in different ratios such that expression of the HIV co-receptor inhibitor will be higher than that of the HIV replication or fusion inhibitor. For instance, the ratio of expression of the first nucleic acid sequence to the second nucleic acid sequence can be from about 2:1 to greater than about 10:1, preferably from about 5:1 to about 10:1, more preferably from about 2:1 to about 5:1. In one embodiment, the ratio of expression of the first nucleic acid sequence to the second nucleic acid sequence is about 2:1. In embodiments in which the expression vector comprises three nucleic acid sequences, the ratio of expression of the first, second, and third nucleic acid sequences can be manipulated such that the expression of HIV co-receptor inhibitors will be higher than that of the HIV replication and fusion inhibitors. For instance, in embodiments in which the first nucleic acid sequence encodes an inhibitor of an HIV co-receptor (*e.g..* CCR5 or CXCR4), the second nucleic acid sequence encodes a fusion inhibitor, and the third nucleic acid sequence encodes a replication inhibitor, the ratio of expression of the first, second, and third nucleic acid sequences can be from about 2:1:1 to about 10:1:1, from about 5:1:1 to about 10:1:1, or from about 2:1:1 to about 5:1:1.

Generation of the expression vectors described herein can be accomplished using any suitable genetic engineering techniques well known in the art, including, without limitation, the standard techniques of PCR, oligonucleotide synthesis, restriction endonuclease digestion, ligation, transformation, plasmid purification, and DNA sequencing, for example as described in Sambrook et al. (Molecular Cloning--A Laboratory Manual (3rd Ed.), Vol. 1-3, Cold Spring Harbor Laboratory, Cold Spring Harbor, N.Y., (2000)), Ausubel et al. (Current Protocols in Molecular Biology, Greene Publishing Associates, Inc. and John Wiley & Sons, Inc.), Coffin et al. (Retroviruses. Cold Spring Harbor Laboratory Press, N.Y. (1997)) and "RNA Viruses: A Practical Approach" (Alan J. Cann, Ed., Oxford University Press, (2000)).

In one embodiment, the expression vector is an FG12 vector, and more preferably an FG11F lentiviral vector (See Example 1). In another embodiment, the second nucleic acid sequence is cloned into two restriction sites (*e.g.,* BamHI and EcoRI of the FG11 F vector). In yet another embodiment, the first nucleic acid sequence is inserted between two restriction sites (*e.g.,* Xbal/Xhol sites of the FG11F vector). In certain embodiments, the viral expression vector further comprises at least one further nucleic acid molecule capable of inhibiting HIV infection, selected from a shRNA or siRNA, an antisense molecule, a ribozyme or an aptamer targeted to a HIV viral sequence or host sequence. In other embodiments, the viral expression vector further comprises one or more protein-encoding nucleic acid sequences as described herein. For instance, in one embodiment, the viral expression vector further comprises one or more nucleic acid sequences encoding protein inhibitors of HIV viral fusion or HIV replication.

The novel expression vectors of the invention confer resistance to infection by more than one HIV variant when expressed in host cells. In one embodiment, the novel expression vectors, when expressed in host cells, confer resistance to infection by R5- and X4-tropic strains of HIV. In some embodiments, when expressed in host cells, the expression vectors confer resistance to infection by strains of HIV that are resistant to HAART or Maraviroc therapies.

The present invention also includes a method of producing a viral expression vector that is capable of inhibiting binding of HIV to the cell and preventing HIV fusion to the cell or HIV replication when expressed in a host cell. In one embodiment, the method comprises synthesizing a cDNA of a gene which expresses a protein capable of preventing HIV fusion into a cell or HIV replication; cloning the synthesized cDNA into a restriction site in a viral vector; and inserting an expression unit capable of downregulating expression of an HIV co-receptor into a restriction site in the vector. The cDNA can be from any gene which expresses any of the protein fusion or replication inhibitors described herein. For instance, in one embodiment, the cDNA is a C46 cDNA. In another embodiment, the cDNA is a TRIM5α cDNA or a cyclophilin fusion thereof. The expression unit capable of downregulating expression of a HIV co-receptor can be any of the inhibitory RNA molecules described herein, such as siRNA, shRNA, or antisense targeting the co-receptor. In one embodiment, the expression unit is a shRNA targeting CCR5. In one particular embodiment, the shRNA targeting CCR5 has a sequence of SEQ ID NO: 1.

The viral vector can be a retroviral vector. In certain embodiments, the viral vector is a lentiviral vector, such as the FG11F lentiviral vector. In some embodiments, the cDNA of a gene encoding a protein fusion or replication inhibitor is cloned into restriction sites BamHI and EcoRI of an FG11F vector. In other embodiments, the expression unit capable of downregulating expression of an HIV co-receptor is inserted between XbaI/XhoI restriction sites of the FG11F vector. Other lentiviral vectors and restriction sites suitable for use in the method are known to those of ordinary skill in the art.

The present invention also provides a host cell comprising the novel expression vectors of the invention. A "host cell" or "target cell" means a cell that is to be transformed using the methods and expression vectors of the invention. In some embodiments, the host cells are mammalian cells in which the expression vector can be expressed. Suitable mammalian host cells include, but are not limited to, human cells, murine cells, non-human primate cells (*e.g*. rhesus monkey cells), human progenitor cells or stem cells, 293 cells, HeLa cells, D17 cells, MDCK cells, BHK cells, and Cf2Th cells. In certain embodiments, the host cell comprising an expression vector of the invention is a hematopoietic cell, such as hematopoietic progenitor/stem cell (*e.g*. CD34-positive hematopoietic progenitor/stem cell (HPSC)), a monocyte, a macrophage, a peripheral blood mononuclear cell, a CD4+ T lymphocyte, a CD8+ T lymphocyte, or a dendritic cell. In some embodiments, the host cell is a CCR5+ hematopoietic cell. In other embodiments, the host cell may be a host cell from a patient or matched to a patient. In certain embodiments, a host cell transduced with the expression vectors of the invention are resistant to infection by X4 or R5 -tropic HIV strains, including HAART resistant strains.

Methods of delivering expression vectors and nucleic acids to cells are known in the art and can include, for example, viral infection, calcium phosphate co-precipitation, electroporation, microinjection, DEAE-dextran, lipofection, transfection employing polyamine transfection reagents, cell sonication, gene bombardment using high velocity microprojectiles, and receptor-mediated transfection.

The present invention also encompasses a pharmaceutical composition comprising the novel expression vectors of the invention. In one embodiment, the pharmaceutical composition comprises an effective amount of at least one of the expression vectors as described herein and a pharmaceutically acceptable carrier. For instance, in certain embodiments, the pharmaceutical composition comprises an effective amount of an expression vector and a pharmaceutically acceptable carrier, wherein said expression vector comprises a first nucleic acid sequence encoding an inhibitor of an HIV co-receptor and a second nucleic acid sequence encoding a protein that inhibits HIV fusion to a target cell or HIV replication, as described herein.

The phrases "pharmaceutically acceptable" or "pharmacologically acceptable" refer to molecular entities and compositions that do not produce adverse, allergic, or other untoward reactions when administered to an animal or a human. As used herein, "pharmaceutically acceptable carrier" includes solvents, buffers, solutions, dispersion media, coatings, antibacterial and antifungal agents, isotonic and absorption delaying agents and the like acceptable for use in formulating pharmaceuticals, such as pharmaceuticals suitable for administration to humans. The use of such media and agents for pharmaceutically active substances is well known in the art. Except insofar as any conventional media or agent is incompatible with the expression vectors of the present invention, its use in therapeutic compositions is contemplated. The pharmaceutical compositions of the invention may be formulated for administration by various routes of administration including, but not limited to, oral, nasal, buccal, intradermal, subcutaneous, intramuscular, intraperitoneal, or intravenous injection. In some embodiments, the pharmaceutical compositions may be formulated as suppositories for rectal administration. Supplementary active ingredients also can be incorporated into the compositions, provided they do not inactivate the vectors or polynucleotides of the compositions.

The pharmaceutical compositions of the present invention may include classic pharmaceutical preparations. By way of illustration, solutions of the active compounds as free base or pharmacologically acceptable salts can be prepared in water suitably mixed with a surfactant, such as hydroxypropylcellulose. Dispersions can also be prepared in glycerol, liquid polyethylene glycols, and mixtures thereof and in oils. Under ordinary conditions of storage and use, these preparations generally contain a preservative to prevent the growth of microorganisms.

The pharmaceutical forms suitable for injectable use include, for example, sterile aqueous solutions or dispersions and sterile powders for the extemporaneous preparation of sterile injectable solutions or dispersions. Generally, these preparations are sterile and fluid to the extent that easy injectability exists. Preparations should be stable under the conditions of manufacture and storage and should be preserved against the contaminating action of microorganisms, such as bacteria and fungi. Appropriate solvents or dispersion media may contain, for example, water, ethanol, polyol (for example, glycerol, propylene glycol, and liquid polyethylene glycol, and the like), suitable mixtures thereof, and vegetable oils. The proper fluidity can be maintained, for example, by the use of a coating, such as lecithin, by the maintenance of the required particle size in the case of dispersion and by the use of surfactants. The prevention of the action of microorganisms can be brought about by various antibacterial and antifungal agents, for example, parabens, chlorobutanol, phenol, sorbic acid, thimerosal, and the like. In many cases, it will be preferable to include isotonic agents, for example, sugars or sodium chloride. Prolonged absorption of the injectable compositions can be brought about by the use in the compositions of agents delaying absorption, for example, aluminum monostearate and gelatin.

Sterile injectable solutions may be prepared by incorporating the active compounds in an appropriate amount into a solvent along with any other ingredients (for example as enumerated above) as desired, followed by filtered sterilization. Generally, dispersions are prepared by incorporating the various sterilized active ingredients into a sterile vehicle which contains the basic dispersion medium and the desired other ingredients, *e.g*., as enumerated above. In the case of sterile powders for the preparation of sterile injectable solutions, the preferred methods of preparation include vacuum-drying and freeze-drying techniques which yield a powder of the active ingredient(s) plus any additional desired ingredient from a previously sterile-filtered solution thereof.

The compositions of the present invention generally may be formulated in a neutral or salt form. Pharmaceutically-aeceptable salts include, for example, acid addition salts (formed with the free amino groups of the protein) derived from inorganic acids (*e.g*., hydrochloric or phosphoric acids), or from organic acids (*e.g*., acetic, oxalic, tartaric, mandelic, and the like). Salts formed with the free carboxyl groups of the protein can also be derived from inorganic bases (*e.g*., sodium, potassium, ammonium, calcium, or ferric hydroxides) or from organic bases (*e.g*., isopropylamine, trimethylamine, histidine, procaine and the like).

The present invention also includes a method of treating or preventing HIV infection in a patient in need thereof. As used herein, "patient" or "subject" may encompass any vertebrate including but not limited to humans and mammals. However, advantageously, the patient or subject is a mammal such as a human or non-human primate, or a mammal such as a domesticated mammal, *e.g*., dog, cat, horse, and the like, or production mammal, *e.g*., cow, sheep, pig, and the like. Where the patient is other than human, the invention provides a method of treating or preventing an HIV-related infection in the patient (*e.g*., infection by SIV, FIV, or BIV). In a preferred embodiment, the patient is a human.

In one embodiment, the method comprises administering a pharmaceutical composition comprising an expression vector of the invention as described herein. For instance, in some embodiments, the method comprises administering to the patient a pharmaceutical composition comprising an expression vector wherein said expression vector comprises a first nucleic acid sequence encoding a shRNA targeting CCR5 (or CXCR4) and a second nucleic acid sequence encoding a C46 protein, and optionally wherein said first nucleic acid sequence is operably linked to a first promoter and said second nucleic acid sequence is operably linked to a second promoter as described. In other embodiments, the method comprises administering to the patient a pharmaceutical composition comprising an expression vector wherein said expression vector comprises a first nucleic acid sequence encoding a shRNA targeting CCR5 (or CXCR4) and a second nucleic acid sequence encoding a TRIM5α protein or derivative or fusion thereof, and optionally wherein said first nucleic acid sequence is operably linked to a first promoter and said second nucleic acid sequence is operably linked to a second promoter as described. In still other embodiments, the method comprises administering to the patient a pharmaceutical composition comprising an expression vector wherein said expression vector comprises a first nucleic acid sequence encoding an inhibitor of an HIV co-receptor (*e.g*., shRNA to CCR5 or CXCR4), a second nucleic acid sequence encoding a fusion inhibitor (*e.g*., C46), and a third nucleic acid sequence encoding an inhibitor of HIV replication (*e.g*., TRIM5α protein or a derivative or fusion thereof), optionally wherein said first, second, and third nucleic acid sequences are operably linked to first, second, and third promoters as described herein. In another embodiment, the method comprises administering to the patient a pharmaceutical composition comprising an expression vector wherein said expression vector comprises a first nucleic acid sequence encoding a first inhibitor of an HIV co-receptor (*e.g*., shRNA to CCR5), a second nucleic acid sequence encoding a second inhibitor of an HIV co-receptor (*e.g*., shRNA to CXCR4), and a third nucleic acid sequence encoding an inhibitor of HIV viral fusion to a target cell or HIV replication, optionally wherein said first, second, and third nucleic acid sequences are operably linked to first, second, and third promoters as described herein.

In certain embodiments, the patient to whom the pharmaceutical composition is administered is a patient at risk of infection by R5 and X4 tropic strains of HIV, including HAART resistant strains, and such risk is ameliorated following administration of the composition. In some embodiments, the patient is HIV negative. In other embodiments, the patient (*e.g*., human) may be HIV positive and naïve to highly active antiretroviral therapy (HAART) -that is the human patient has never received HAART, which includes combinations of nucleoside analogue reverse transcriptase inhibitors, protease inhibitors, and non-nucleoside reverse transcriptase inhibitors. In some embodiments, the patient is receiving a HAART regimen. In still other embodiments, the patient is failing or has failed on a HAART regimen (*i.e*. HAART is ineffective in reducing viral load due to resistance). Thus, in certain embodiments, the expression vector is introduced directly to the patient either prophylatically for a patient who is HIV negative or to treat a patient who is HIV positive.

The expression vectors of the compositions can be modified such that they are specifically localized to particular cell types, such as immune cells. By way of example, the expression vector may be combined with a receptor-mediated gene targeting vehicle, wherein said targeting vehicle comprises a cell-receptor-specific ligand and a DNA-binding agent. Alternatively, a cell receptor-specific ligand can be attached to a liposome comprising the expression vector The cell-receptor-specific ligands can be chosen depending on the cell types of interest. For instance, in some embodiments, the expression vector may be localized to CD34+ cells by employing a ligand that binds to the CD34 cell surface marker. One of skill in the art can choose appropriate ligands to target specific cell types, such as immune cells (*e.g*., a monocyte/macrophage, a peripheral blood mononuclear cell, a CD4+ T lymphocyte, a CD8+ T lymphocyte, or a dendritic cell). In certain embodiments in which the expression vector is a viral vector, the viral vector can be packaged in viral particles having a particular tropism for certain cell types. For example, in one embodiment, the viral vector is packaged in HIV retroviral particles thereby allowing the recombinant retrovirus to infect CD4+ T cells and macrophages.

Administration to a patient of the pharmaceutical compositions according to the present invention may be via any common route so long as the target tissue is available via that route. This includes oral, nasal, or buccal. Alternatively, administration may be by intradermal, subcutaneous, intramuscular, intraperitoneal or intravenous injection. In one embodiment, the pharmaceutical composition may be administered rectally (*e.g*., with a suppository). Upon formulation, solutions are preferably administered in a manner compatible with the dosage formulation and in such amount as is therapeutically effective. The formulations may easily be administered in a variety of dosage forms such as injectable solutions, drug release capsules and the like. For parenteral administration in an aqueous solution, for example, the solution generally is suitably buffered and the liquid diluent first rendered isotonic for example with sufficient saline or glucose. Such aqueous solutions may be used, for example, for intravenous, intramuscular, subcutaneous and intraperitoneal administration. Preferably, sterile aqueous media are employed as is known to those of skill in the art particularly in light of the present disclosure. By way of illustration, a single dose may be dissolved in 1 ml of isotonic NaCl solution and either added to 1000 ml of hypodermoclysis fluid or injected at the proposed site of infusion, (see for example, "Remington's Pharmaceutical Sciences" 15th Edition, pages 1035-1038 and 1570-1580). Some variation in dosage may occur depending on the stage of HIV infection in the patient being treated. The person responsible for administration will, in any event, determine the appropriate dose for the individual patient. Moreover, for human administration, preparations should meet sterility, pyrogenicity, general safety and purity standards as required by FDA Office of Biologics standards.

In another embodiment, the present invention provides a method of treating or preventing HIV infection in a patient by administering to the patient HIV-resistant hematopoietic cells produced by transducing the cells with an expression vector of the invention. For example, in one embodiment, the method comprises transducing hematopoietic cells *ex vivo* with an expression vector described herein, and infusing the transduced cells into the patient. One or more infusions of the transduced cells can be administered to the patient. In some embodiments, the patient receives multiple infusions of the transduced cells over a periodic interval, such as weekly, biweekly, monthly, quarterly, or annually. In one embodiment, the patient receives an infusion of the transduced cells every two weeks. Hematopoietic cells suitable for use in the method include, but are not limited to, hematopoietic progenitor/stem cells (HPSC), monocytes, macrophages, peripheral blood mononuclear cells, CD4+ T lymphocytes, CD8+ T lymphocytes, and dendritic cells. In one embodiment, the hematopoietic cells used in the method are CD4+ T lymphocytes, CD8+ T lymphocytes, or monocyte/macrophages. In a preferred embodiment, the hematopoietic cells used in the method are HPSC. As used herein, transduced hematopoietic cells include the transduced cells themselves as well as cells derived from the transduced cells (*e.g*., cells generated from transduced HPSC).

Thus, in one particular embodiment, the present invention provides a method of treating or preventing HIV infection in a patient by reconstituting the immune system with HIV-resistant cells generated from transduced HPSC. For instance, in one embodiment, the method comprises transducing HPSC with an expression vector as described herein and transplanting said transduced HPSC in the patient, wherein said transplanted cells generate granulocytes, monocyte/macrophages, and lymphocytes that are resistant to HIV infection. The granulocytes, monocyte/macrophages, and lymphocytes are resistant to infection by R5 and X4 tropic strains of HIV. In some embodiments, the granulocytes, monocyte/macrophages, and lymphocytes are resistant to infection by HIV strains that are resistant to HAART. The patient can be HIV negative or HIV positive. In one embodiment, the human patient is naïve to highly active antiretroviral therapy (HAART) In another embodiment, the patient is receiving a HAART regimen. In yet another embodiment, the patient is failing or has failed on a HAART regimen.

The hematopoietic cells (*e.g*. HPSC, CD4+ T lymphocytes, CD8+ T lymphocytes, and/or monocyte/macrophages) to be transduced with an expression vector of the invention can be allogeneic or autologous. "Allogeneic cells" refer to cells obtained from different individuals of the same species. As used herein, the phrase "autologous cells" refers to cells isolated from a patient that are subsequently reimplanted or injected into the same patient. Thus, an autologous transplantation is one in which the donor and recipient are the same patient. In certain embodiments, the hematopoietic cells are autologous HPSC. The HPSC are preferably CD34-positive and can be isolated from the patient's bone marrow or peripheral blood. Methods for such purification are known to those in the art (see, for example, U.S. Patent Nos.: 4,965,204, 4,714,680, 5,061,620, 5,643,741, 5,677,136, 5,716,827, 5,750,397, and 5,759,793). For instance, one method for purifying such CD34-positive stem cells involves centrifugation of peripheral blood samples to separate mononuclear cells and granulocytes, followed by fluorescence activated cell sorting (FACS) to select CD34+ cells. In one embodiment, the cells are enriched for CD34+ cells through a magnetic separation technology such as that available from Miltenyi Biotec and that has been previously described (Kogler et al. (1998) Bone Marrow Transplant., Vol. 21:223-241; Pasino et al. (2000) Br. J. Haematol., Vol. 108: 793-800). CD34-positive cells may be mobilized from the marrow into the blood prior to collection by injecting the patient with one or more cytokines known to mobilize hematopoietic stem cells, such as granulocyte colony stimulating factor, granulocyte-macrophage stimulating factor, and stem cell factor.

The isolated CD34-positive HPSC (and/or other hematopoietic cell described herein) is preferably transduced with an expression vector of the invention. For example, in one embodiment the expression vector comprises a first nucleic acid sequence encoding an inhibitor of an HIV co-receptor and a second nucleic acid sequence encoding a protein that inhibits HIV fusion to a target cell or HIV replication, optionally wherein said first nucleic acid sequence is operably linked to a first promoter and said second nucleic acid sequence is operably linked to a second promoter. In another embodiment, the expression vector comprises a first nucleic acid sequence encoding an inhibitor of an HIV co-receptor, a second nucleic acid sequence encoding a fusion inhibitor, and a third nucleic acid sequence encoding an inhibitor of HIV replication, optionally wherein said first, second, and third nucleic acid sequences are operably linked to first, second, and third promoters. In still another embodiment, the expression vector comprises a first nucleic acid sequence encoding a first inhibitor of an HIV co-receptor, a second nucleic acid sequence encoding a second inhibitor of an HIV co-receptor, and a third nucleic acid sequence encoding an inhibitor of HIV viral fusion to a target cell or HIV replication, optionally wherein said first, second, and third nucleic acid sequences are operably linked to first, second, and third promoters.

In one embodiment, the first nucleic acid sequence (or second nucleic acid sequence in embodiments in which the expression vector comprises three nucleic acid sequences) encodes a siRNA or shRNA having a double-stranded region, wherein the double-stranded region comprises a sequence that is substantially identical and complementary to a sequence of CCR5. In another embodiment, the first nucleic acid sequence encodes a shRNA targeting CCR5 that has a sequence of SEQ ID NO: 1. In another embodiment, the first nucleic acid sequence encodes a siRNA or shRNA having a double-stranded region, wherein the double-stranded region comprises a sequence that is substantially identical and complementary to a sequence of CXCR4. In particular embodiments, the transduced hematopoietic cells (*e.g.* HPSC, CD4+ T lymphocytes, CD8+ T lymphocytes, and/or monocyte/macrophages) or cells generated from them express reduced levels of a HIV co-receptor (*e.g*. CCR5 or CXCR4) protein as compared to non-transduced hematopoietic cells. For instance the transduced hematopoietic cells or cells generated from them may express 30%, 40%, 50%, 60%, 65%, 75%, 80%, 85%, 90%, or 95% less HIV co-receptor protein as compared to non-transduced hemapoietic cells. In other embodiments, the hematopoietic cells are transduced with an expression vector of the invention in which the second nucleic acid sequence (or third nucleic acid sequence in embodiments in which the expression vector comprises three nucleic acid sequences) encodes a TRIM5α protein or derivative or fusion thereof, such as human TRIM5 α rhesus TRIM5 α, chimeric TRIM5 α, or a human TRIM5-cyclophilin fusion protein. In still other embodiments, the hematopoietic cells are transduced with an expression vector of the invention in which the second nucleic acid sequence (or third nucleic acid sequence in embodiments in which the expression vector comprises three nucleic acid sequences) encodes a C46 protein. In such embodiments, the transduced hematopoietic cells or cells generated from them express increased levels of the protein (*e.g.* C46 or TRIM5α or derivative or fusion thereof) as compared to non-transduced hematopoietic cells, that is 20%, 30%, 40%, 50%, 60%, 70%, 80%. 90%, 100%, 150%, or greater than 200% more of the encoded protein as compared to non-transduced hematopoietic cells.

Following transduction of the hematopoietic cells (*e.g.,* HPSC, CD4+ T lymphocytes, CD8+ T lymphocytes, or monocyte/macrophages) with an expression vector of the invention, the transduced cells are reintroduced or transplanted back into the patient. The transduced cells can be injected parenterally into the patient, or reintroduced by any other route known in the art. In one embodiment, the transduced hematopoietic cells are injected intravenously into the patient. Preferably, an effective dose of transduced hematopoietic cells is administered to the patient. An "effective dose" is an amount sufficient to effect a beneficial or desired clinical result and can depend on the type of hematopoietic cell used. In one embodiment, the hematopoietic cell is a HPSC and an effective dose is an amount that is sufficient to at least partially reconstitute the immune system with HIV-resistant cells. Said dose could be administered in one or more administrations and may be from about 0.5 x 10⁶ HPSC per kg patient weight to about 1 x 10⁹ HPSC per kg patient weight. In another embodiment, the hematopoietic cell is a CD4+ T lymphocyte, a CD8+ T lymphocyte, or a monocyte/macrophage and an effective dose may be from about 1 x 10⁹ cells per patient to 1 x 10¹¹ cells per patient. However, the precise determination of what would be considered an effective dose may be based on factors individual to each patient, including their size, age, severity of HIV infection (*e.g.* viral titer), and amount of time since contraction of the virus. One skilled in the art, specifically a physician, would be able to determine the number of transduced hematopoietic cells which would constitute an effective dose without being subjected to undue experimentation.

Without being bound by theory. Applicants believe that a successful stem cell therapy for HIV disease includes selection for transduced, engrafted cells. In one aspect of the invention, detailed kinetic studies on HIV infected individuals demonstrate that HIV-1 kills and the body replenishes approximately 10⁹ to 10¹⁰ CD4+ T-cells each day. This represents a turnover of 0.5% to 5% of the total CD4+ T-cell population each day resulting in an estimated turnover of the entire CD4+ T-cell population approximately every 2 weeks. Therefore, even in healthy untreated HIV infected individuals, a stable CD4+ T-cell count masks massive ongoing death and replenishment of T-cells. T-cells are replaced from two sources-expansion of existing peripheral T-cells, and production of new naïve T-cells derived from the thymus, in a manner similar to that seen in generation of new T-cells following HPSC transplant. The present invention provides a method to reconstitute with gene-modified HPSC that provide a continual source of protected T-cells and monocyte/macrophages. These cells are likely to be selected for in the face of massive HIV T-cell depletion.

The concept of utilizing selective pressures involving T-cell death and regeneration to select for gene-transduced cells is based upon a solid foundation of knowledge in hematopoietic and lymphoid differentiation. The concept has also been tested successfully in the gene therapy clinical studies for X-linked SCID and ADA-SCID where gene containing T-cells derived from transplanted HPSC are similarly selected resulting in repopulation with genetically modified T-cells.

A recent case study provides support that reconstitution of an immune system with cells protected from HIV-1 infection can result in selection for the protected cells, substantial attenuation of HIV-1 replication and a favorable clinical course. An HIV-1 positive individual with concurrent AML was treated by transplant of allogeneic HPSC specifically chosen from a CCR5Δ32 homozygous donor. Remarkably, the CCR5Δ32 donor cells completely replaced the recipient cells within a rapid 60 days and the patient has remained undetectable for HIV-1 for more than 200 days in the absence of anti-retroviral therapy.

The invention will now be illustrated in greater detail by reference to the specific embodiments described in the following examples. The examples are intended to be purely illustrative of the invention and are not intended to limit its scope in any way.

### EXAMPLES

### Example 1. Construction of Dual Vector Containing shRNA against CCR5 and C46 Fusion Inhibitor (sh5/C46 Dual Vector) and Control Vectors with Single or no Therapeutic Inserts

### A. Vector Plasmid Constructs

A variety of constructs were designed and engineered in the DNA form as plasmids. The constructs are summarized in Table 1 and illustrated in Figures 1-4. All of these constructs give rise to lentiviral vectors upon transfection into packaging cell lines (see section B below).

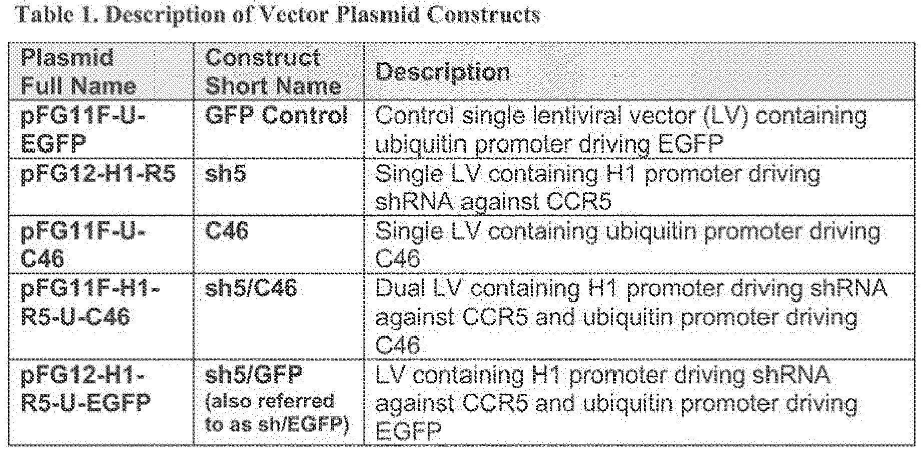

The genetic engineering of these vectors was as follows.

The pFG12 backbone lentiviral vector plasmid containing EGFP driven by the ubiquitin promoter (pFG12-U-EGFP) (labeled as "pFG12" in Figure 2) was derived from an earlier lentiviral vector FUGW (Lois et al. (2002) Science, Vol. 295: 868-872) as described (Qin et al. (2003) Proc. Natl. Acad. Sci., Vol. 100: 183-188). To aid further insertions into the backbone vector, the plasmid backbone pFG11F was produced by inserting multiple cloning sites into FG12, enabling production of pFG11F-U-EGFP (labeled as "pG11F" in Figure 2).

A small hairpin RNA (shRNA) random library directed against human chemokine co-receptor 5 (huCCR5) under the control of an H1 promoter within a lentiviral vector was produced via enzymatic production of RNAi libraries from c-DNAs. The purified DNA fragments were digested with BpmI, blunt-ended with Klenow fragment, digested with BamHI and ligated to pBShH1-5 plasmid DNA, which contains a human H1 RNA polymerase III promoter and 4T termination signal. The ligation mixture was introduced into *E. coli* and plated overnight. Colonies were combined and plasmid DNA prepared. shRNA expression units consisting of an H1 promoter, shRNA sequence and 4Ts termination signal were excised from the pBShH1-5 plasmid DNAs by XbaI and XholI digestion and inserted into XbaI/XholI sites of the pFG12-U-EGFP vector to produce H1 promoter driven shRNA against CCR5. The best of these constructs, sh1005, was selected for further experimentation. The plasmid construct containing sh1005 and ubiquitin promoter-driven EGFP is termed pFG12-H1-R5-U-EGFP (Figure 3; An et al. (2007) Proc. Natl. Acad. Sci., Vol. 104 (32): 13110-13115). The U-EGFP cassette was removed from pFG12-H1-R5-U-EGFP using restriction enzymes to produce pFG12-H1-R5 (Figure 3).

The EGFP gene was removed from pFG11F-U-EGFP (pFG11F in Figure 4) and replaced with the C46 gene to produce pFG11F-U-C46 (Figure 4). The H1-R5 cassette was excised from pFG12-H1-R5-U-EGFP using an NdeI / XhoI digest and inserted into pFG11F-U-C46, which had also been digested with NdeI / XhoI, to produce pFG11F-H1-R5-U-C46 (Figure 4).

### B. Lentiviral Vector Production

All vesicular stomatitis virus (VSV)-G pseudotyped lentiviral vector stocks were produced by calcium phosphate-mediated transient transfection of HEK-293 T cells. HEK-293 T cells were routinely cultured in DMEM (GIBCO Invitrogen) and changed to Iscove's modified Dulbecco's medium (IMDM) for transfection. All cultures contained 10% FCS (HyClone), 100 units of penicillin, and 100 µg/ml streptomycin. The cells were co-transfected with appropriate amounts of vector plasmid, the HIV-1 lentiviral packaging constructs pRSV-Rev and pMDLg/pRRE, and the VSV-G expression plasmid pCMV-VSV-G (Table 2). The viruses were collected from the culture supematants on days 2 and 3 post-transfection and concentrated. The concentrated virus stocks were titered on HEK-293 T cells based on GFP expression. Titers for the shRNA expression EGFP constructs were only slightly reduced compared with the parental EGFP vector. The plasmids used for production are shown diagrammaticalty in Figure 5.

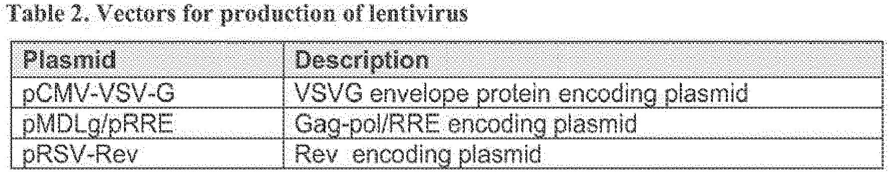

### Method 1: Lentivirus production by calcium chloride transfection using non-kit reagents

1. HEK 293T cells seeded at 1.5x10⁷ cells per T175 flask in DMEM + 10% FBS and antibiotics the day prior to transfection.
2. On the day of transfection, medium changed to 25 mL IMDM with 10% FBS, antibiotics and chloroquine(100 µl of 10 mM).
3. DNA master mix prepared
   a. pMDLg /pRRE 10 µg
   b. pRSV-Rev 2.5 µg
   c. pCMV-VSV-G 3.2 µg
   d. vector (e.g. one of the constructs from Figs. 1-4) 10 µg
   e. Water added to adjust total volume to 980 µL
4. 133 µL 2M CaCl₂ added, mixed and incubated on ice for 10 min.
5. 1110 µL 2 x HBS (1g Hepes, 1.6g NaCl, 0.72ml 0.25M Na₂HPO₄, 1ml 1M KCl) added drop by drop while shaking the tube by hand.
6. Incubated on ice for 20min.
7. T175 flask of cells tipped upside down, DNA mixture added to medium, flask mixed 2-3 times and flask flipped right way up.
8. Culture incubated 6-8h
9. Medium removed and replaced with fresh 42mL IMDM + 10% FBS and antibiotics.
10. 48h post transduction, medium harvested and filtered through 0.22 or 0.45µM filter and replaced with fresh 42ml IMDM + 10% FBS and antibiotics.
11. 72h post transduction, medium harvested and filtered through 0.22 or 0.45µM filter.
12. Both harvests are pooled
13. Virus containing medium (VCM) concentrated by ultracentrifugation in SW28 or SW32 tubes.
   a. 33-38 mL VCM loaded into tubes with sucrose cushion.
   b. Tubes centrifuged at 20,000 rpm at 4°C for 90 min.
   c. Supernatant removed and 250-500 µL PBS or HBS added to pellet.
   d. Store VCM overnight at 4°C.
   e. VCM mixed by pipetting, aliquoted and stored at -70°C.

### Method 2: Lentivirus production by calcium chloride transfection using Clontech CalPhos Kit.

1. HEK 293T cells seeded at 2.1x10⁷ cells/T225 in 30 mL IMDM + 10% FBS on the day prior to transfection.
2. On the day of transfection, DNA master mix prepared in 15 mL tubes:
   a. pMDLg /pRRE 13 µg
   b. pRSV-Rev 3.25 µg
   c. pCMV-VSV-G 4.16 µg
   d. vector (*e.g.* one of the constructs from Figs. 1-4) 13µg
   e. Water added to adjust total volume to 1500 µL
3. 186 µL 2M CaCl₂ added and mixed.
4. 1500 µL 2 x HBS added drop by drop while vortexing the tube.
5. Incubated at room temperature for 20 min.
6. 30 mL IMDM + 2% FBS added to 50 mL tube.
7. DNA solution added to IMDM in 50 mL tube.
8. Media aspirated from cells harvested the previous day.
9. DNA/IMDM solution gently poured into flask so as not to disturb cell monolayer.
10. Flask gently rocked from side to side to cover cells with mixture.
11. Culture incubated for 4 hours.
12. Medium removed, cells rinsed with PBS and replaced with fresh 30 mL IMDM + 2% FBS.
13. At 24 hours post transduction, medium harvested and replaced with fresh 30 mL IMDM + 2% FBS.
14. Harvested VCM filtered through 0.22 µM filter and stored at 4°C overnight.
15. At 48 hours post transduction, medium harvested and filtered through 0.22 µM filter.
16. Both VCM collections pooled and aliquoted for storage at -70°C.
17. If required, VCM concentrated using Vivaspin 20 (Sartorius) columns:
   a. Vivaspin 20 MWCO 100 000 prepared by adding 10 mL 70% ethanol
   b. Spun at 1000g for 10 min.
   c. Remaining ethanol discarded and 15 mL PBS added.
   d. Spun at 1000g for 10 min.
   e. Remaining PBS discarded and 18 mL VCM added.
   f. Spun 1000g for 30 min or until all VCM has passed through the column.

The VCM obtained by either method was used (diluted or concentrated) to transduce target cells (T cell lines, peripheral blood mononuclear cells (PBMC), CD34+ hematopoietic progenitor stem cells (HPSC)) and the transduced cells were analyzed by flow cytometry for EGFP expression, CCR5 expression (via CD195 antibody staining) and C46 expression (via 2F5 antibody staining).

### Example 2. Transduction of Human Target T Cell Lines by sh5/C46 Dual Vector

The various lentiviral vectors described in Example 1 were used to infect CEM.NKR.CCR5 and Molt4/CCR5 cells (NIH AIDS Reagent Program) cells. 2 x 10⁵ cells were resuspended in 1mL unconcentrated virus containing medium (VCM) with 10% FBS and 8 µg/mL polybrene. Cultures were incubated at 37°C for 1.5 hours and a further 1 mL of growth media added (RPMI + 10% FBS). Cells were analyzed by FACS analysis 4 days post transduction for C46 expression (by 2F5 antibody staining), CCR5 knockdown (by CD195 antibody staining), and GFP expression. Cells were kept in continuous culture for up to 8 weeks by passaging twice weekly.

Simultaneous expression of shRNA (detected by CCR5 knockdown) and C46 in transduced CEM.NKR.CCR5 and Molt4/CCR5 cells is shown in Figure 6 and Figure 7, respectively. GFP expression was observed for the constructs containing EGFP (panels 1,3 from left to right); a reduction in CCR5 expression (*e.g*. down-modulation of CCR5 showing expression of shRNA) was observed for the constructs containing sh5 (panels 2,3,5, from left to right), and C46 expression (as measured by 2F5 antibody) was observed for the constructs containing C46 (panels 4,5, from left to right). Percentage positive cells are shown in each flow cytometry quadrant (Q1-Q4) for each group of cells transduced with the indicated lentiviral vectors at 4 and 8 weeks in culture. Similar expression levels were seen at weeks 4 and 8. Mean Fluorescence Intensity (MFI) Values for Figure 6 are shown in Table 3 below, while MFI values for Figure 7 are shown in Table 4 below.

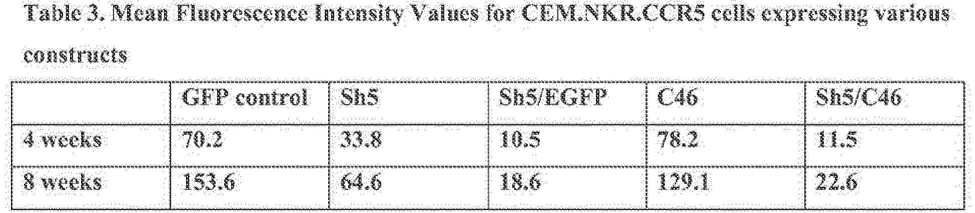

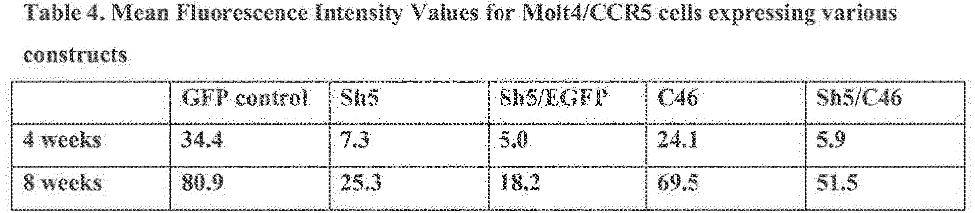

To determine if the transgenes caused any differences in the growth parameters of the cells, CEM.NKR.CCR5 cells, which each showed 100% expression of the transgenic construct, were seeded at 2 x 10⁴ cells/mL, cultured for 4 days and counted. Cells were then seeded from this population on four separate occasions over a 3 week period at 1 or 2 x 10⁵/mL and counted 4-7 days later. No differences were observed in the growth rates of the cells transduced with the different constructs (Figure 8).

The results of these experiments show that both the shRNA targeting CCR5 and the C46 protein can be expressed sufficiently from the same vector in human T cell lines, and expression of CCR5 shRNA and C46 has no effect on the growth rate of the cells.

### Example 3. Transduction of Human Peripheral Blood Mononuclear Cells (PBMC) by sh5/C46 Dual Vector

The various lentiviral vectors described in Example 1 were used to infect human peripheral blood mononuclear cells (PBMC) obtained from the Australian Red Cross Blood Transfusion Service. PBMC were isolated from bufty coats using Ficoll-plaque PLUS (GE Healthcare) followed by CD8 depletion using CD8+ Microbeads (Miltenyi Biotec) and a VarioMACS magnetic unit. CD8+ depleted PBMC were cultured for 48 hours in RPMI 1640 media supplemented with 20% FBS and 5 µg/mL phytohemagglutinin (PHA) (Sigma) at 2 x 10⁶ cells/mL. Following 2 days PHA stimulation, cells in suspension were harvested, centrifuged at 200g for 5 minutes and resuspended at 2x10⁶ cells/mL in RPMI + 20% FBS + 10 U/mL recombinant human interleukin-2 (rhIL-2; Roche) for 4 hours prior to transduction.

To ascertain the preferred transduction method, PBMC were transduced with the sh5/EGFP lentiviral construct using various conditions: 1X transduction with VCM, 2X transduction with VCM, 1X transduction with VCM preload (Preload 1), 2X transduction with VCM preload (Preload 2), concentrated VCM approximately 20-fold (concentrated)(see Example 1, Section B). As shown in Figure 9, transduction was most efficient with concentrated virus. A single transduction with VCM preload (Preload 1) was chosen as the preferred method for further experiments. MFI values for Figure 9 are shown in Table 5 below.

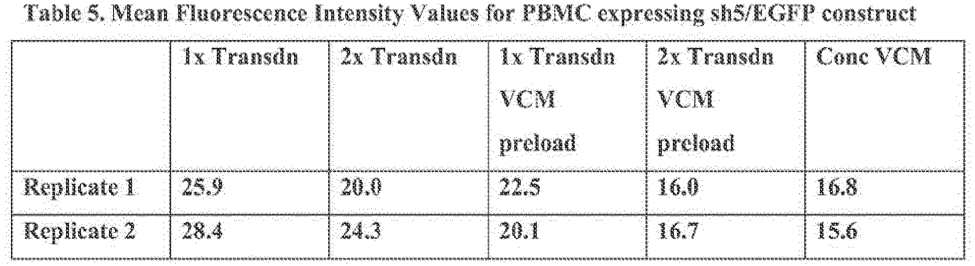

PBMC were either left untransduced or transduced (1X pre-load) with one of sh5/GFP, C46, sh5/C46, GFP control or sh5 lentiviral constructs according to the following procedure. 1mL PBMC were transferred onto Retronectin coated 24-well plates (5 µg/cm²) previously preloaded (6 hours) with 250 µL of unconcentrated VCM and cultured overnight. The following day, cells were transferred to 6-well plates in 3mL RPMI + 20% FBS + 10 U/mL rhIL-2. Cells were analyzed for EGFP, CCR5 and C46 expression 4 days post transduction. The results are shown in Figures 10-13.

As shown in Figure 10, expression of EGFP, CCR5, and C46 in PBMC at day 4 post transduction was as expected for the different constructs. EGFP expression was observed for the constructs containing EGFP (GFP control and sh5/GFP; panels 1,2); a reduction in CCR5 expression (illustrating expression of CCR5 shRNA) was seen for the constructs containing sh5 (sh5/EGFP and sh5/C46, panels 2 and 4), and C46 expression (as measured by 2F5 antibody) was observed for the constructs containing C46 (C46 and sh5/C46; panels 3,4). MFI values, from left to right in Figure 10, were 16.2, 8.4, 16.8, 9.4.

Figure 11 shows a comparison of gene expression in transduced PBMC (at day 4) and transduced CEM.NKR.CCR5 T cell line (at week 8). EGFP expression was observed in cells transduced with the constructs containing EGFP (GFP control and sh5/GFP; panels 1 and 2); CCR5 down-regulation was seen in cells transduced with the constructs containing sh5 (sh5/EGFP and sh5/C46; panels 2 and 4), and C46 expression (as measured by 2F5 antibody) was observed in cells transduced with the constructs containing C46 (C46 and sh5/C46; panels 3 and 4). Although sufficient levels of expression from the lentiviral constructs were observed in both cell types, higher levels of expression were observed in the T cells as compared to PBMC. MFI values for Figure 11 are shown in Table 6 below.

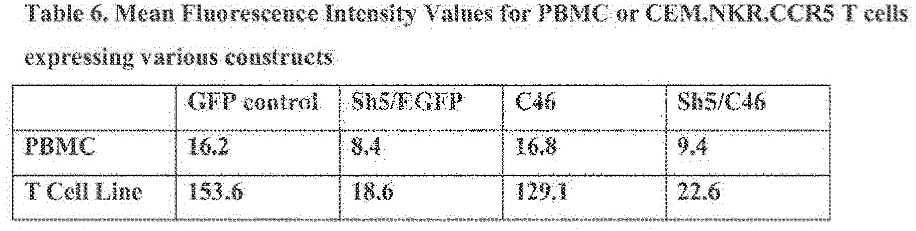

In addition, growth rates were compared between gene-transduced (sh5, sh5/EGFP, C46, sh5/C46) and non-transduced PBMC at days 1, 4, 8 and 12. Two replicate seeds of each group were used. The total cells/well and percentage of viable cells were similar for all transduced PBMC when compared to one another and to the untransduced cells (Figure 12).

Stability of transgene expression in PBMC was also tested. Figure 13 shows expression of EGFP, CCR5, and C46 (as measured by 2F5 antibody) in cells transduced with the indicated constructs at days 4, 7 and 12. Viability of cells at day 12 was uncertain and therefore comparisons were made between days 4 and 7 only. As shown in Figure 13, the various transgenes were expressed at both time points with an apparent decline over time, which is probably related to decreasing growth and viability over time (see Figure 12). MFI values for Figure 13 are shown in Table 7 below.

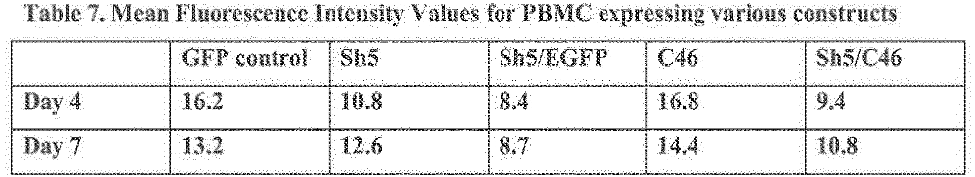

These results show that both the shRNA targeting CCR5 and the C46 protein can be expressed sufficiently from the same vector in human PBMC.

### Example 4. Transduction of Human Hematopoietic Progenitor/Stem Cells (HPSC) by sh5/C46 Dual Vector

The sh5/C46 lentiviral vector (LV) was used to transduce CD34+ hematopoietic progenitor/stem cells (HPSC) obtained from bulk donor peripheral blood mononuclear cells. Donors were injected with granulocyte colony-stimulating factor (G-CSF) to mobilize HPSC and peripheral blood mononuclear cells. Following G-CSF injection, the cells were harvested by apheresis and the bulk mononuclear cell population containing mobilized HPSC were frozen. The mononuclear cell sample used in this example was obtained from these frozen stocks. A 50 mL stem cell harvest bag estimated at the time of freezing to contain 3.7 x 10⁷ CD34+ HPSC, was thawed. On thawing, it was found to contain a total of 33.6 x 10⁸ viable cells (73% viability) and the resulting number of CD34+ HPSC isolated using MACS (Magnetic Antibody Cell Separation) was within expectations at ∼3.3 x 10⁷ i.e. ∼1% of the total mononuclear cell number, with 98% CD34 positive (See pre- and post-separation analysis in upper panel of Figure 14).

These cells were then used in the following experimental protocol:
1. 6 x 10⁶ cells were pre-stimulated for 24 hours in X-vivo serum free media containing Stem Cell Factor (SCF), thrombopoietin (TPO) and Flt3 ligand (Flt3L) (each at 50 ng/mL).
2. Aliquots of 4 x 10⁵ cells were then transferred to a 12-well plate preloaded for 6 hours with Virus Containing Medium (VCM). The cells were transduced overnight (with GFP control, sh5, sh5/EGFP, C46, or sh5/C46), or left untransduced, and then transferred to fresh media for 72 hours.
3. FACS analysis performed 72 hours post-transduction demonstrated 25-30% transduction by GFP (Figure 14, bottom panel). In this example, C46 was undetectable by 2F5 staining apparently due to the lack of sensitivity of the flow cytometry with these cells. The remaining cells were put into CAMEO-4 (Hemogenix) methylcellulose cultures, plated at 100 cells/well in replicate. When scored for colonies, no significant differences were seen between the cultures at day 11 (Table 8).

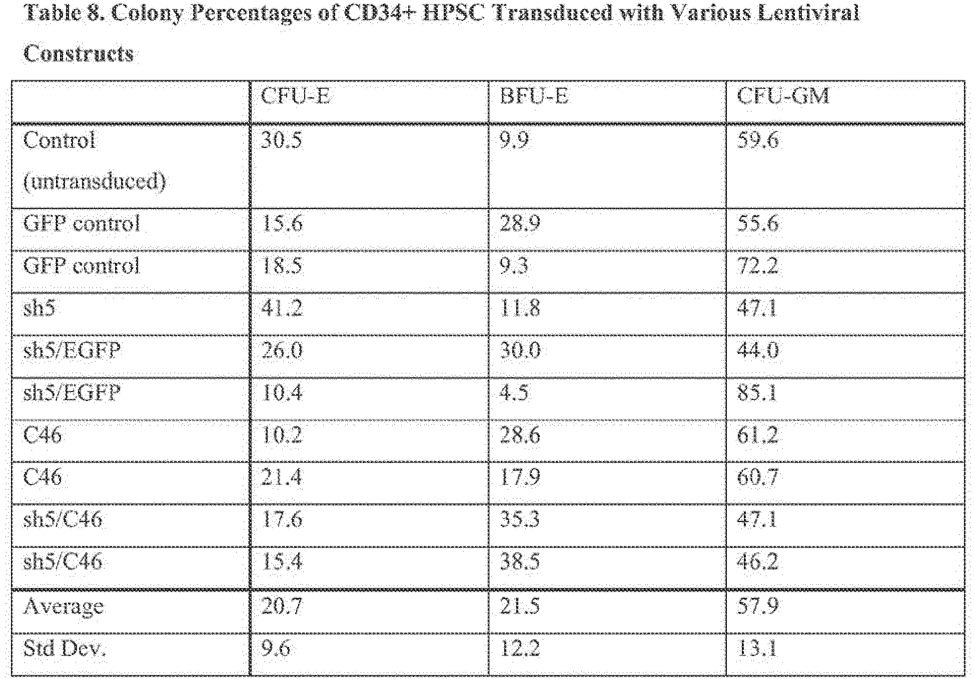

### Example 5. sh5/C46 Dual Vector-Trausduced T Cell Lines Inhibit HIV Replication

T cell lines (Molt4/CCR5) transduced with the sh5/C46 dual lentiviral construct (see description of vector in Example 1) were challenged with various strains of HIV: HIV_{Bal} (CCR5 tropic), HIV_{HIB}, (CXCR4 tropic), and HIV_{SF2} (CCR5 and CXCR4 tropic). For challenge assays, 1 x 10⁶ transduced Molt4/CCR5 cells were added to 15 mL tubes and centrifuged. The supernatant was discarded. HIV virus containing medium (VCM) was added to a final concentration per tube at a multiplicity of infection (MOI) of 0.2-0.002. Polybrene was then added to a final concentration of 8 µg/mL and each tube was tapped gently. Cells and virus were incubated for 2 hours at 37°C with gentle shaking every 30 minutes. Following the 2 hour incubation, cells were washed in media (RPMI + 10% FBS) and resuspended in 3-4 mL of media in T25 flasks. Cells were sampled and fed every 3-4 days until day 11. 150 µl of supernatant was removed in duplicate and stored at 4°C. P24 protein levels (a measure of HIV infection) was assayed as per Manufacturers' protocol generally using 1/10⁵ to 1/10⁶ dilution to ensure values were on Standard Curve.

Figure 15 shows the p24 protein levels from non-transduced cells or cells transduced with the dual sh5/C46 lentiviral construct 13 days following challenge with dual tropic HIV strain SF2 (CCR5 and CXCR4 tropic). The results show that cells transduced with the sh5/C46 construct exhibited an approximate 2 log inhibition at all three MOIs (0.2. 0.02, 0.002) in each of 2 independent samplings as compared to non-transduced cells. Figure 16 shows the p24 protein levels from non-transduecd cells or cells transduced with either the sh5/C46 or C46 lentiviral construct 11 days following challenge with dual tropic HIV strain SF2. The data show approximately 2 log inhibition by sh5/C46 construct in each of two independent samplings and 3 log inhibition by C46 (apparently due to higher expression of C46 in this particular construct) at the two MOIs tested. The bottom panel of Figure 16 shows expression by flow cytometry. Mean Fluorescence Intensity values are shown in Table 9 below.

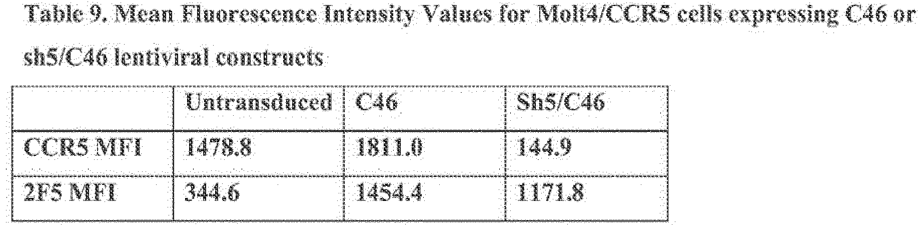

In a separate experiment Molt4/CCR5 cells were either non-transduced or transduced with C46 (Gene 2) or sh5/C46 (G2R5) lentiviral constructs and subsequently challenged with mV-SF2 dual tropic (CCR5 and CXCR4), Bal (CCR5 tropic) or NL4-3 (CXCR4 tropic) virus at an MOI of 0.2. P24 protein levels were assessed 11 days after viral challenge as a measure of HIV infection. As shown in Figure 17, cells expressing both lentiviral constructs were effective in reducing infection with all three strains of HIV. Figure 18 shows p24 protein levels from non-transduced cells (Molt4) or cells transduced with one of four lentiviral constructs [(1) sh5 (R5); (2) C46 (G2); (3) sh5/C46 (R5-G2); (4) sh5/EGFP (R5-GFP)] 7 and 10 days following challenge with CCR5 tropic HIV strain Bal at a MOI of 0.2. The "mix" group is a mixture of untransduced, sh5, C46, sh5/C46 all mixed equally (*i.e*. 25% of each). The results show that cells expressing the shRNA against CCR5 and the C46 gene from a single lentiviral construct (dual construct) provide enhanced protection against infection with a CCR5 tropic HIV strain at both 7 and 10 days following viral challenge.

The results of this series of experiments show that T cells transduced with the dual sh5/C46 lentiviral construct provide protection against infection with CCR5, CXCR4, and dual tropic CCR5 and CXCR4 HIV strains.

### Example 6. sh5/C46 Dual Vector-Transduced PBMC Inhibit HIV Replication

Phytobemagglutinin (PHA)/IL2-stimulated peripheral blood mononuclear cells (PBMC) were transduced with lentiviral vectors as described in Example 3. A schematic of the dual construct expressing shRNA against CCR5 and C46 protein (LVsh5C46) is shown in Figure 19A. Four days post-transduction, cells were stained with the appropriate monoclonal antibody (*e.g*., CD195 or 2F5 antibody) and CCR5, C46, and GFP expression was analyzed by flow cytometry (Figure 19B). Lentiviral (LV)- transduced PBMC were challenged with R5 or X4 tropic HIV strains 16 days after LV transduction. Culture supernatants were collected four days after HIV infection and assayed for p24 protein by ELISA (Figure 19C).

As shown in Figure 19C, PBMC transduced with sh5/C46 lentiviral vector exhibit reduced HIV infection induced by both R5 and X4 tropic strains as assessed by p24 protein levels. PBMC transduced with a sh5/GFP construct are resistant to infection induced by R5 but not X4 tropic HIV. These results indicate that the sh5/C46 dual vector is capable of protecting against HIV infection induced by either R5 or X4 tropic strains.

### Example 7. sh5 Vector Down Regulates CCR5 Expression in Lymphoid Organs and Effects Preferential Survival of Transduced CD4+ T Lymphocytes Ex Vivo

sh5 lentiviral-transduced CD34+ hematopoietic progenitor/stem cells (HPSC) solidified with Matrigel in combination with a thymus segment were implanted under the kidney capsule of a humanized bone marrow/liver/thymus (BLT) mouse model (see Melkus et al. (2006) Nat Med, Vol. 12:1316-1322; Shimizu et al. (2010) Blood, Vol. 115:1534-1544). The NOD/SCID-hu BLT humanized mouse allows examination of the differentiation of transduced human HPSC in the human thymus-like organoid (thy/liv), and migration of differentiated human T lymphocytes in systemic lymphoid organs including gut associated lymphoid tissue - the major site of HIV replication.

To examine sh1005 (shRNA targeting CCR5) in this humanized mouse model, vector-transduced fetal liver-derived CD34+ cells and CD34- cells solidified with matrigel and a thymus segment were transplanted under the kidney capsule to generate a vector-transduced thy/liv tissue. Three weeks later, vector-transduced autologous CD34+ HPSC (1 x 10⁶ cells) were injected through the tail vein of the sub-lethally irradiated mouse. To assess the impact of CCR5 reduction within an animal, an equal mix of sh1005 vector (EGFP+)- and non-shRNA control vector (mCherry+)-transduced CD34+ HPSC (5 x 10⁵ cells) were co-transplanted. This experimental design allows one to examine whether sh 1005 vector-transduced cells differ from non-shRNA vector-transduced cells with regard to level of stability and specificity of CCR5 reduction; both vectors are present within the same animal to control for mouse to mouse variation. Neither EGFP nor mCherry alone had any effect on repopulation kinetics or CCR5 expression (data not shown).

Human cell engraftment was examined from 11 weeks post-CD34+ injection. Human CD45+ lymphoid cells were detected in a gated lymphocyte population of peripheral blood from transplanted mice by flow cytometric analysis (average 44%, SD±28, n=19). EGFP and mCherry expression was found in this human CD45+ population in transplanted mice (average EGFP 22%, SD±19, average mCherry 22%, SD±13, n=16). CCR5-knockdown in human CD4+ and CD45+ T-lymphocytes in various lymphoid tissues in reconstituted animals at 14-20 weeks post CD34+ HPSC transplant was examined (Figure 20A). CCR5 expression was efficiently reduced in EGFP+ human CD4+ and CD45+ T-lymphocytes in all tissues analyzed. Notably, CCR5 reduction was efficient even in the highly CCR5-expressing lamina propria lymphocytes isolated from the gut. CCR5 was not reduced in mCherry+ human CD4+/CD45+ T-lymphocytes in the same animal. These results indicate that the CCR5-shRNA expression did not affect human T-lymphocyte differentiation and migration and effectively induced CCR5 down-regulation in systemic lymphoid organs *in vivo.*

To examine HIV susceptibility in CCR5 down-regulated cells, EGFP+ and mCherry+ splenocytes were isolated from the animal by cell sorting. The sorted cells were infected with either R5 tropic HIV-1_{NFNSXSL9} or X4 tropic HIV-1_{NL4-3} at a multiplicity of infection of 2.5 in triplicate. There was no increase in p24 HIV gag capsid protein production in the culture supernatant of EGFP+ splenocytes over the 12 day culture period (Figure 20B). In contrast, mCherry+ splenocytes were susceptible to R5 tropic HIV-1_{NFNSXSL9}, and produced approximately 4-fold higher levels of p24 in the culture supernatant on days 7 and 12 (P value=0.003), indicating CCR5 downregulation effectively inhibited R5 tropic HIV-1 infection. In contrast to the R5 tropic HIV-1 infection, X4 tropic HIV-I_{NL4-3} infection produced comparable amounts of p24 in both EGFP+ and mCherry+ splenocyte culture supernatants confirming the specificity of the inhibition (P value=0.23). These results demonstrated that downregulation of CCR5 by sh1005 was sufficient to protect *ex vivo* stimulated cells from R5 but not X4 tropic HIV-1 challenge.

To examine HIV susceptibility *in vivo* and selective protection and survival of CCR5 down-regulated CD4+ T-cells, R5 tropic HIV-1_{NFNSXSL9} was injected intravenously (p24=200 ng) into reconstituted mice at 9 weeks post HPSC transplant. Mice were confirmed to be infected with HIV by the presence of p24 in supernatant of mouse peripheral blood co-cultured with PHA/IL2 activated human PBMC at 8 week post HIV injection. The kinetics of EGFP and mCherry expressing CD4+ T-cells in peripheral blood were examined (Figure 20C). Percent EGFP+ population in CD4+ T-cells in peripheral blood increased from 20% to 40% by 8 week post HIV injection in the animals. In contrast, the mCherry+ population in CD4+ T-cells declined from 40% to 3% in the animals. The inversion of CD4/CD8 ratio indicating HIV induced CD4 T-cell loss was examined. The CD4/CD8 ratio in EGFP+ lymphocytes was maintained above 1 by 8 week post HIV challenge in peripheral blood (Figure 20D). In contrast, CD4/CD8 ratio in mCherry+ CD45+ cells was inverted to 0.1, These results demonstrate stable CCR5 down-regulation by sh1005 was sufficient to preferentially increase CD4+ T-cells following R5-tropic HIV challenge *in vivo.*

### Example 8. Testing of sh5/C46 Dual Vector in Humanized Mouse Model

As described in Example 7 for the sh5 vector (Shimizu et al. (2010) Blood, Vol. 115: 1534-1544), the sh5/C46 dual lentiviral vector is tested in the humanized BLT mouse model. To examine the sh5/C46 dual vector in this humanized mouse model, vector-transduced fetal liver-derived CD34+ cells and CD34- cells solidified with matrigel and a thymus segment are transplanted under the kidney capsule to generate a vector-transduced thy/liv tissue. Three weeks later, vector-transduced autologous CD34+ HPSC (1 x 10⁶ cells) are injected through the tail vein of the sub-lethally irradiated mouse. To assess the impact of CCR5 reduction and C46 expression within an animal, an equal mix of the dual sh5/C46 vector (EGFP+)- and control (empty lentiviral) vector (mCherry+)-transduced CD34+ HPSC (5 x 10⁵ cells) are co-transplanted. Other controls, such as sh5 single vector containing another fluorescent protein (*e.g*. YFP) and C46 single vector containing yet another fluorescent protein (*e.g* CFP) are used to transduce CD34+ HPSC and are present in the mixture for transplantation. This experimental design allows one to examine the difference between cells transduced with various constructs with regard to level of stability and specificity of CCR5 reduction and C46 expression; all vectors are present within the same animal to control for mouse to mouse variation.

Control (mCherry+) and active sh5/C46 (EGFP+) transduced cells are compared over time using flow cytometry and RT-PCR. Comparisons are made between sh5/C46-transduced cells and cells transduced with one of the single vectors (sh5 or C46). Susceptibility to HIV infection is examined by injecting an R5, X4, or dual tropic HIV strain intravenously into reconstituted animals following HPSC transplant. Percent of CD4+c T-cells and ratios of CD4/CD8 T-cells in each of the vector-transduced populations is assessed to ascertain the effectiveness of CCR5 knockdown and C46 expression on CD4+ T cell survival.

### Example 9. Use of Dual Constructs in Human HIV Patients

A dual lentiviral construct, including the sh5/C46 dual vector, the sh5/TRIM5α dual vector, or the sh5/TRIM5α-cyclophilin dual vector, is introduced into autologous human cells and subsequently provided to the patient. The dual lentiviral construct is introduced into one or more of CD34+ HPSC cells, CD4+ T-cells, CD8+ T-cells, monocyte/macrophages isolated from the patient to whom they will be re-implanted (*e.g*. autologous cells). Alternatively cells from another individual (allogeneic) are used. Alternatively a triple vector as described herein is used.

With respect to HIV tropism, many patients will have R5 virus, a smaller proportion will have X4 virus, and an intermediate number will have a mixed population. The dual constructs described herein have the ability to target both R5 and X4 virus and can be beneficial for patients with a mixed cell population and may also prevent resistance in those with a single population. The constructs can also be beneficial in patients with HAART resistant virus.

The cells for transduction are obtained from the patient by injecting one or more cytokines that mobilize HPSC and other cells, and the relevant cell populations are separated for lentiviral transduction. The transduced-cells are intravenously introduced into the same patient or another patient in order to treat or prevent HIV infection. One or more doses or infusions of the transduced cells are used as described herein.

The clinical trial is designed based on considerations including the patient's clinical condition, previous treatment and/or resistance to treatment. Different patient groups are included in the trial. For example, one subset of patients has not yet received highly active antiretroviral therapy (*i.e.* naive to HAART). In general, these patients are quite healthy (notwithstanding their background HIV infection) and selection criteria for receiving dual lentiviral-transduced hematopoietic cells may include those patients who have a history of a relatively rapid CD4 decline, high viral load, and/or early symptoms. Figure 21 shows an expected response in such a patient group. Dual lentiviral vector-transduced cells are introduced to a patient at time 0. Figures 21A and 21B show predictions of viral load and CD4 count in patients treated with one infusion of transduced cells (star) versus patients not receiving dual lentiviral vector-transduced cells (triangle). The untreated patients are expected to maintain a high viral load and a continuing decrease in CD4 count over time. In contrast, those treated with the dual lentiviral vector-transduced cells are expected to show viral load decrease over time and CD4 count increase (after a potential initial small drop due to apheresis). Thus the treatment may delay the need for HAART and/or decrease its requirement once HAART is initiated.

A second subset of patients is HIV positive and currently well-controlled on HAART. Figure 22 details an expected response to a single infusion of dual lentiviral vector-transduced cells in such a patient group. Predicted viral load for a patient treated with one dose of transduced cells (star) versus an untreated patient (triangle) is shown. Dual lentiviral-vector-transduced cells are introduced to a patient at time 0. Two HAART treatment interruptions are undertaken at various time points (ATI), *e.g*. from weeks 24-28 and 40-48, with the patients staying off HAART if viral load remains below a pre-set safety limit (*e.g*., 100K copies/mL). The HAART interruptions are to provide a period where there can be HIV-induced preferential survival of those cells protected by the dual lentiviral construct and a resulting decrease in viral load. Primary end-point is at week 48 but one can also measure area under the viral load curve from weeks 40-48 and 40-100. Predicted viral load decreases in the long-term for both treated and untreated patients (though more slowly for patients not receiving dual lentiviral-transduced cell infusions) as the patients go back on HAART (as required). The treatment may decrease the need for HAART and its associated complications.

A third patient group comprises individuals who are failing HAART because of resistance to HAART drugs, non-compliance, or some other reason. Figure 23 depicts predicted viral load (Figure 23A) and predicted CD4 count (Figure 23B) expected in such a patient. After infusion of dual lentiviral-transduced cells at day 0, viral load is expected to decrease and CD4 count to increase (star) as compared to an untreated patient where viral load is expected to remain the same or increase and CD4 count is expected to decrease with time (triangle).

Endpoints in all of the patient groups include viral load, CD4 counts, time to resumption/initiation of HAART, transduced cell percentage, and T-Cell Receptor Excision Circles (measure of recent thymic emigrants) and decreased requirement for HAART.

### Example 10. Construction of Dual Vector Containing shRNA against CCR5 and TRIM5a (sh5/TRIM5a Dual Vector)

A dual lentiviral vector containing an shRNA targeting CCR5 under the control of a H1 promoter and a nucleic acid encoding a TRIM5α protein under the control of a ubiquitin promoter is constructed using the backbone vectors described in Example 1. For example, the U-EGFP cassette is removed from pFG12-H1-R5-U-EGFP, the plasmid construct containing sh1005 and ubiquitin promoter-driven EGFP (see Figure 3), using restriction enzymes to produce pFG12-H1-R5.

The EGFP gene is removed from pFG11F-U-EGFP (pFG11F in Figure 4) and is replaced with the TRIM5α gene (SEQ ID NO: 5) to produce pFG11F-U-TRIM5α. The H1-R5 cassette is excised from pFG12-H1-R5-U-EGFP using an NdeI / XhoI digest and is inserted into pFG11F-U- TRIM5α, which has also been digested with NdeI / XhoI, to produce pFG11F-H1-R5-U- TRIM5α (Figure 24A). This construct is used to make lentivirus as described in Section B of Example 1.

### Example 11. Construction of Triple Vector Containing shRNA against CCR5, C46, and TRIM5a (sh5/ C46/TRIM5a Triple Vector)

A triple vector is produced from the dual vector pFG11F-H1--R5-U-C46 by cloning β-actin promoter-TRIM5α into a multicloning site as shown in Figure 24B.

All publications, patents and patent applications discussed and cited herein are incorporated herein by reference in their entireties. It is understood that the disclosed invention is not limited to the particular methodology, protocols and materials described as these can vary. It is also understood that the terminology used herein is for the purposes of describing particular embodiments only and is not intended to limit the scope of the present invention which will be limited only by the appended claims.

Those skilled in the art will recognize, or be able to ascertain using no more than routine experimentation, many equivalents to the specific embodiments of the invention described herein. Such equivalents are intended to be encompassed by the following claims.

The following numbered paragraphs contain statements of broad combinations of the inventive technical features herein disclosed :
[Paragraph 1] An expression vector comprising a first nucleic acid sequence encoding an inhibitor of an HIV co-receptor and a second nucleic acid sequence encoding a protein that inhibits HIV fusion to a target cell or HIV replication.
[Paragraph 2] The expression vector of paragraph 1, wherein the expression vector is a viral vector.
[Paragraph 3] The expression vector of paragraph 2, wherein the viral vector is a lentiviral vector or a retroviral vector.
[Paragraph 4] The expression vector of paragraph 3, wherein the lentiviral vector is self-inactivating.
[Paragraph 5] The expression vector of paragraph 1, wherein the expression vector confers resistance to infection by X4- and R5-tropic HIV strains when expressed in a host cell.
[Paragraph 6] The expression vector of paragraph 1, wherein the expression vector confers resistance to infection by highly active antiretroviral therapy (HAART)-resistant HIV strains when expressed in a host cell.
[Paragraph 7] The expression vector of paragraph 1, further comprising a third nucleic acid sequence encoding an inhibitor of HIV viral binding, HIV viral fusion to the target cell membrane or HIV viral replication.
[Paragraph 8] The expression vector of paragraph 1, wherein the inhibitor of an HIV co-receptor is a siRNA or shRNA having a double-stranded region, wherein the double-stranded region comprises a sequence that is substantially identical and complementary to a sequence of said HIV co-receptor.
[Paragraph 9] The expression vector of paragraph 8, wherein said HIV co-receptor is CCR5 or CXCR4.
[Paragraph 10] The expression vector of paragraph 9, wherein the shRNA has a sequence of SEQ ID NO: 1.
[Paragraph 11] The expression vector of paragraph 1, wherein the inhibitor is capable of reducing expression of the HIV co-receptor when said vector is expressed in a host cell.
[Paragraph 12] The expression vector of paragraph 1, wherein the protein that inhibits HIV fusion to a target cell is a C46 protein, a T20 protein, enfuvirtide, CP₃₂M, and sifuvirtide.
[Paragraph 13] The expression vector of paragraph 1, wherein the protein that inhibits HIV replication is selected from the group consisting of human TRIM5α, rhesus TRIM5α, chimeric TRIM5α, a human TRIM5-cyclophilin fusion protein, cyclophilin, E3 ubiquitin, APOBEC3G, and bone marrow stromal cell antigen 2 (BST-2).
[Paragraph 14] The expression vector of paragraph 13, wherein the chimeric TRIM5α comprises an amino terminal domain from a human TRIM5α protein and a carboxy terminal PRYSPRY domain from a rhesus TRIM5α protein.
[Paragraph 15] The expression vector of paragraph 1, wherein said first and second nucleic acid sequences are operably linked to a promoter.
[Paragraph 16] The expression vector of paragraph 15, wherein said promoter is a RNA polymerase II or RNA polymerase III promoter.
[Paragraph 17] The expression vector of paragraph 1, wherein said first nucleic acid sequence is operably linked to a first promoter and said second nucleic acid sequence is operably linked to a second promoter.
[Paragraph 18] The expression vector of paragraph 17, wherein said first and second promoters are the same.
[Paragraph 19] The expression vector of paragraph 17, wherein said first and second promoters are different.
[Paragraph 20] The expression vector of paragraph 19, wherein said first promoter is a RNA polymerase III promoter and wherein said second promoter is a RNA polymerase II promoter.
[Paragraph 21] The expression vector of paragraph 7, wherein said third nucleic acid sequence is operably linked to a third promoter.
[Paragraph 22] The expression vector of paragraph 21, wherein said third promoter is the same as or different from the first and second promoters.
[Paragraph 23] The expression vector of paragraph 16, wherein said RNA polymerase III promoter is an H1 pol III promoter.
[Paragraph 24] The expression vector of paragraph 16, wherein said RNA polymerase II promoter is a UbiquitinC pol II promoter.
[Paragraph 25] A host cell comprising the expression vector of paragraph 1.
[Paragraph 26] The host cell of paragraph 25, wherein the host cell is resistant to infection by X4- or R5-tropic HIV strains.
[Paragraph 27] The host cell of paragraph 26, wherein the host cell is resistant to infection by HAART-resistant HIV strains.
[Paragraph 28] The host cell of paragraph 25, wherein said host cell is a hematopoietic progenitor/stem cell, a monocyte, a macrophage, a peripheral blood mononuclear cell, a CD4+ T lymphocyte, a CD8+ T lymphocyte, or a dendritic cell.
[Paragraph 29] An expression vector comprising a first nucleic acid sequence encoding an shRNA having a sequence of SEQ ID NO: 1 and a second nucleic acid sequence encoding for a C46 protein, wherein said first nucleic acid sequence is operably linked to an H1 pol III promoter and said second nucleic acid sequence is operably linked to a UbiquitinC pol II promoter.
[Paragraph 30] An expression vector comprising a first nucleic acid sequence encoding a first shRNA, a second nucleic acid sequence encoding a second shRNA, and a third nucleic acid sequence encoding an inhibitor of HIV viral fusion to a target cell or HIV replication.
[Paragraph 31] The expression vector of paragraph 30, wherein said first nucleic acid sequence is operably linked to a first promoter, said second nucleic acid sequence is operably linked to a second promoter, and said third nucleic acid sequence is operably linked to a third promoter.
[[Paragraph 32] The expression vector of paragraph 30, wherein said first shRNA targets CCR5 and said second shRNA targets CXCR4.
[Paragraph 33] A method of treating or preventing HIV infection in a patient comprising transducing hematopoietic cells with the expression vector of paragraph 1 and transplanting said transduced hematopoietic cells in the patient, wherein said transduced hematopoietic cells are resistant to HIV infection.
[Paragraph 34] The method of paragraph 33, wherein said hematopoietic cells are hematopoietic progenitor/stem cells (HPSC), CD4+ T lymphocytes, CD8+ T lymphocytes, monocyte/macrophages, or combinations thereof.
[Paragraph 35] The method of paragraph 34, wherein said transplanted HPSC generate granulocytes, monocyte/macrophages, and lymphocytes that are resistant to HIV infection.
[Paragraph 36] The method of paragraph 33, wherein said hematopoietic cells are autologous or allogeneic.
[Paragraph 37] The method of paragraph 33, wherein said first nucleic acid sequence encodes a siRNA or shRNA having a double-stranded region, said double-stranded region comprising a sequence that is substantially identical and complementary to a sequence of CCR5.
[Paragraph 38] The method of paragraph 37, wherein the shRNA has a sequence of SEQ ID NO: 1.
[Paragraph 39] The method of paragraph 37, wherein said transduced hematopoietic cells express reduced levels of CCR5 protein as compared to non-transduced hematopoietic cells.
[Paragraph 40] The method of paragraph 33, wherein said second nucleic acid sequence encodes a protein selected from the group consisting of human TRIM5α, rhesus TRIM5α, chimeric TRIM5α, a human TRIM5-cyclophilin fusion protein and a C46 protein.
[Paragraph 41] The method of paragraph 40, wherein the chimeric TRIM5α comprises an amino terminal domain from a human TRIM5α protein and a carboxy terminal PRYSPRY domain from a rhesus TRIM5α protein.
[Paragraph 42] The method of paragraph 40, wherein said transduced hematopoietic cells express increased levels of said protein as compared to non-transduced hematopoietic cells.
[Paragraph 43] The method of paragraph 35, wherein said granulocytes, monocyte/macrophages, and lymphocytes are resistant to infection by R5 and X4 tropic strains of HIV.
[Paragraph 44] The method of paragraph 43, wherein said granulocytes, monocyte/macrophages, and lymphocytes are resistant to infection by HAART-resistant HIV strains.
[Paragraph 45] The method of paragraph 33, wherein the patient is naïve to HAART.
[Paragraph 46] The method of paragraph 33, wherein the patient is receiving a HAART regimen.
[Paragraph 47] The method of paragraph 33, wherein the patient is failing or has failed on a HAART regimen.
[Paragraph 48] A pharmaceutical composition comprising an effective amount of the expression vector of paragraph 1 and a pharmaceutically acceptable carrier.
[Paragraph 49] Use of a pharmaceutical composition of paragraph 48 in the manufacture of a medicament for the treatment of a patient infected with HIV.
[Paragraph 50] A method of treating or preventing HIV infection in a patient comprising administering the pharmaceutical composition of paragraph 48 to the patient.
[Paragraph 51] The method of paragraph 50, wherein the patient is resistant to infection by R5 and X4 tropic strains of HIV following administration of the composition.
[Paragraph 52] The method of paragraph 51, wherein the patient is resistant to infection by HAART-resistant HIV strains following administration of the composition.
[Paragraph 53] The method of paragraph 50, wherein the patient is naïve to HAART.
[Paragraph 54] The method of paragraph 50, wherein the patient is receiving a HAART regimen.
[Paragraph 55] The method of paragraph 50, wherein the patient is failing or has failed on a HAART regimen.
[Paragraph 56] The method of paragraph 50, wherein the patient is at risk for HIV infection.
[Paragraph 57] Use of a viral expression vector of paragraph 1 in the manufacture of a medicament for the treatment of a patient infected with HIV.
[Paragraph 58] A method of producing a viral expression vector which, when present in a cell, is
   capable of inhibiting binding of HIV to the cell and preventing HIV fusion into the cell or HIV replication, the method comprising:
   synthesizing a cDNA of a gene which expresses a protein capable of preventing
      HIV fusion into a cell or HIV replication;
   cloning the synthesized cDNA into a restriction site in a viral vector; and
   inserting an expression unit capable of down regulating expression of an HIV co-receptor
      into a restriction site in the vector.
[Paragraph 59] The method of paragraph 58, wherein the cDNA is a C46 cDNA or TRIM5α cDNA, and the expression unit is a shRNA targeting CCR5.
[Paragraph 60] The method of paragraph 59, wherein the shRNA has a sequence of SEQ ID NO: 1.
[Paragraph 61] The method of paragraph 59, wherein the viral vector is a FG11F lentiviral vector.
[Paragraph 62] The method of paragraph 61, wherein the C46 or TRIM5α cDNA is cloned into restriction sites BamHI and EcoRI of an FG11F vector.
[Paragraph 63] The method of paragraph 62, wherein the shRNA is inserted between Xbal/Xhol restriction sites of the FG11F vector.

## Claims

1. A host cell prepared by transducing a hematopoietic cell with an expression vector comprising a first nucleic acid sequence operably linked to a first promoter and a second nucleic acid sequence operably linked to a second promoter, wherein the first nucleic acid sequence encodes an inhibitory nucleic acid molecule that reduces expression of an HIV co-receptor, wherein the second nucleic acid sequence encodes an HIV fusion inhibitor protein, and wherein the second promoter is a UbiquitinC pol II promoter and the HIV fusion inhibitor protein is a C46 protein.

2. The host cell of claim 1, wherein the expression vector is a viral vector.

3. The host cell of claim 2, wherein the viral vector is a lentiviral vector or retroviral vector.

4. The host cell of claim 3, wherein the retroviral vector is self-inactivating.

5. The host cell of any one of claims 1-4, wherein the C46 protein comprises an amino acid sequence having at least 90% sequence identity to SEQ ID NO: 2.

6. The host cell of any one of claim 1-5, wherein the first promoter is an RNA polymerase III promoter.

7. The host cell of claim 6, wherein the RNA polymerase III promoter is an H1 promoter.

8. The host cell of any one of claims 1-7, wherein the HIV co-receptor is CCR5.

9. The host cell of claim 8, wherein the inhibitory nucleic acid molecule is a small interfering RNA (siRNA) or short hairpin RNA (shRNA) having a sequence that is substantially complementary to at least a portion of a nucleic acid sequence encoding the CCR5 co-receptor.

10. The host cell of claim 9, wherein the shRNA comprises the sequence of SEQ ID NO: 1.

11. The host cell of any one of claims 1-10, further comprising a third nucleic acid sequence encoding an inhibitor of HIV replication.

12. The host cell of claim 11, wherein the inhibitor of HIV replication is selected from the group consisting of human TRIM5α, rhesus TRIM5α, chimeric TRIM5α, human TRIM5-cyclophilin fusion protein, cyclophilin, E3 ubiquitin, APOBEC3G, and bone marrow stromal cell antigen 2 (BST-2).

13. The host cell of claim 11 or 12, wherein the third nucleic acid sequence is operably linked to a third promoter.

14. The host cell of any one of claims 1-13, wherein the hematopoietic cell is hematopoietic progenitor/stem cell (HPSC), CD4+ T lymphocyte, CD8+ T lymphocyte, monocyte/macrophage, or combinations thereof.

15. The host cell of any one of claims 1-14, wherein the host cell is resistant to HIV infection.

16. A pharmaceutical composition comprising the host cell of any one of claims 1-15 and a pharmaceutically acceptable excipient or carrier.

17. The host cell of any one of claims 1-15 for use in the treatment of a patient infected with HIV, or a patient at risk for HIV infection.


SEQUENCE LISTING

<110>  CalImmune Inc.
       Chen, Irvin
       An, Dong Sung
       Millington, Michelle
       Boyd, Maureen
       Symonds, Geoff
       Breton, Louis

<120>  DUAL VECTOR FOR INHIBITION OF HUMAN IMMUNODEFICIENCY VIRUS

<130>  CALI-004/01WO

<150>  US 61/225,687
<151>  2009-07-15

<150>  US 61/287,599
<151>  2009-12-17

<160>  5

<170>  PatentIn version 3.5

<210>  1
<211>  51
<212>  RNA
<213>  Unknown

<220>
<223>  shRNA targeting CCR5 receptor

<400>  1
gagcaagcuc aguuuacacc uuguccgacg guguaaacug agcuugcucu u                51


<210>  2
<211>  135
<212>  PRT
<213>  Artificial Sequence

<220>
<223>  C46 HIV fusion inhibitor

<400>  2

<210>  3
<211>  408
<212>  DNA
<213>  Artificial Sequence

<220>
<223>  C46 HIV fusion inhibitor

<400>  3
atgggagcag gagcaaccgg aagggcaatg gacggaccac ggctgctgct gctgctgctg      60

ctgggcgtga gcctgggcgg cgcccggagc tggatggagt gggaccggga gatcaacaac     120

tacaccagcc tgatccacag cctgatcgag gagagccaga accagcagga gaagaacgag     180

caggagctgc tggagctgga caagtgggcc agcctgtgga actggttccg gagcgagcgg     240

aagtgctgcg tggagtgccc accatgccca gcaccaccag tggcaggacc cctgatcgca     300

ctggtgacca gcggagccct gctggccgtg ctgggcatca caggctactt cctgatgaac     360

cggcggagct ggagccccac cggcgagcgg ctggagctgg agccctga                  408

<210>  4
<211>  493
<212>  PRT
<213>  Homo sapiens

<400>  4

Met Ala Ser Gly Ile Leu Val Asn Val Lys Glu Glu Val Thr Cys Pro
1               5                   10                  15

Ile Cys Leu Glu Leu Leu Thr Gln Pro Leu Ser Leu Asp Cys Gly His
            20                  25                  30

Ser Phe Cys Gln Ala Cys Leu Thr Ala Asn His Lys Lys Ser Met Leu
        35                  40                  45

Asp Lys Gly Glu Ser Ser Cys Pro Val Cys Arg Ile Ser Tyr Gln Pro

50                  55                  60

Glu Asn Ile Arg Pro Asn Arg His Val Ala Asn Ile Val Glu Lys Leu
65                  70                  75                  80

Arg Glu Val Lys Leu Ser Pro Glu Gly Gln Lys Val Asp His Cys Ala
                85                  90                  95

Arg His Gly Glu Lys Leu Leu Leu Phe Cys Gln Glu Asp Gly Lys Val
            100                 105                 110

Ile Cys Trp Leu Cys Glu Arg Ser Gln Glu His Arg Gly His His Thr
        115                 120                 125

Phe Leu Thr Glu Glu Val Ala Arg Glu Tyr Gln Val Lys Leu Gln Ala
    130                 135                 140

Ala Leu Glu Met Leu Arg Gln Lys Gln Gln Glu Ala Glu Glu Leu Glu
145                 150                 155                 160

Ala Asp Ile Arg Glu Glu Lys Ala Ser Trp Lys Thr Gln Ile Gln Tyr
                165                 170                 175

Asp Lys Thr Asn Val Leu Ala Asp Phe Glu Gln Leu Arg Asp Ile Leu
            180                 185                 190

Asp Trp Glu Glu Ser Asn Glu Leu Gln Asn Leu Glu Lys Glu Glu Glu
        195                 200                 205

Asp Ile Leu Lys Ser Leu Thr Asn Ser Glu Thr Glu Met Val Gln Gln
    210                 215                 220

Thr Gln Ser Leu Arg Glu Leu Ile Ser Asp Leu Glu His Arg Leu Gln
225                 230                 235                 240

Gly Ser Val Met Glu Leu Leu Gln Gly Val Asp Gly Val Ile Lys Arg
                245                 250                 255

Thr Glu Asn Val Thr Leu Lys Lys Pro Glu Thr Phe Pro Lys Asn Gln
            260                 265                 270

Arg Arg Val Phe Arg Ala Pro Asp Leu Lys Gly Met Leu Glu Val Phe
        275                 280                 285

Arg Glu Leu Thr Asp Val Arg Arg Tyr Trp Val Asp Val Thr Val Ala
    290                 295                 300

Pro Asn Asn Ile Ser Cys Ala Val Ile Ser Glu Asp Lys Arg Gln Val
305                 310                 315                 320

Ser Ser Pro Lys Pro Gln Ile Ile Tyr Gly Ala Arg Gly Thr Arg Tyr
                325                 330                 335

Gln Thr Phe Val Asn Phe Asn Tyr Cys Thr Gly Ile Leu Gly Ser Gln
            340                 345                 350

Ser Ile Thr Ser Gly Lys His Tyr Trp Glu Val Asp Val Ser Lys Lys
        355                 360                 365

Thr Ala Trp Ile Leu Gly Val Cys Ala Gly Phe Gln Pro Asp Ala Met
    370                 375                 380

Cys Asn Ile Glu Lys Asn Glu Asn Tyr Gln Pro Lys Tyr Gly Tyr Trp
385                 390                 395                 400

Val Ile Gly Leu Glu Glu Gly Val Lys Cys Ser Ala Phe Gln Asp Ser
                405                 410                 415

Ser Phe His Thr Pro Ser Val Pro Phe Ile Val Pro Leu Ser Val Ile
            420                 425                 430

Ile Cys Pro Asp Arg Val Gly Val Phe Leu Asp Tyr Glu Ala Cys Thr
        435                 440                 445

Val Ser Phe Phe Asn Ile Thr Asn His Gly Phe Leu Ile Tyr Lys Phe
    450                 455                 460

Ser His Cys Ser Phe Ser Gln Pro Val Phe Pro Tyr Leu Asn Pro Arg
465                 470                 475                 480

Lys Cys Gly Val Pro Met Thr Leu Cys Ser Pro Ser Ser
                485                 490

<210>  5
<211>  1482
<212>  DNA
<213>  Homo sapiens

<400>  5
atggcttctg gaatcctggt taatgtaaag gaggaggtga cctgccccat ctgcctggaa      60

ctcctgacac aacccctgag cctggactgc ggccacagct tctgccaagc atgcctcact     120

gcaaaccaca agaagtccat gctagacaaa ggagagagta gctgccctgt gtgccggatc     180

agttaccagc ctgagaacat acggcctaat cggcatgtag ccaacatagt ggagaagctc     240

agggaggtca agttgagccc agaggggcag aaagttgatc attgtgcacg ccatggagag     300

aaacttctac tcttctgtca ggaggacggg aaggtcattt gctggctttg tgagcggtct 360

caggagcacc gtggtcacca cacgttcctc acagaggagg ttgcccggga gtaccaagtg 420

aagctccagg cagctctgga gatgctgagg cagaagcagc aggaagctga agagttagaa 480

gctgacatca gagaagagaa agcttcctgg aagactcaaa tacagtatga caaaaccaac 540

gtcttggcag attttgagca actgagagac atcctggact gggaggagag caatgagctg 600

caaaacctgg agaaggagga ggaagacatt ctgaaaagcc ttacgaactc tgaaactgag 660

atggtgcagc agacccagtc cctgagagag ctcatctcag atctggagca tcggctgcag 720

gggtcagtga tggagctgct tcagggtgtg gatggcgtca taaaaaggac ggagaacgtg 780

accttgaaga agccagaaac ttttccaaaa aatcaaagga gagtgtttcg agctcctgat 840

ctgaaaggaa tgctagaagt gtttagagag ctgacagatg tccgacgcta ctgggttgat 900

gtgacagtgg ctccaaacaa catttcatgt gctgtcattt ctgaagataa gagacaagtg 960

agctctccga aaccacagat aatatatggg gcacgaggga caagatacca gacatttgtg 1020

aatttcaatt attgtactgg catcctgggc tctcaaagta tcacatcagg gaaacattac 1080

tgggaggtag acgtgtccaa gaaaactgct tggatcctgg gggtatgtgc tggcttccaa 1140

cctgatgcaa tgtgtaatat tgaaaaaaat gaaaattatc aacctaaata cggctactgg 1200

gttatagggt tagaggaagg agttaaatgt agtgctttcc aggatagttc cttccatact 1260

ccttctgttc ctttcattgt gcccctctct gtgattattt gtcctgatcg tgttggagtt 1320

ttcctagact atgaggcttg cactgtctca ttcttcaata tcacaaacca tggatttctc 1380

atctataagt tttctcactg ttctttttct cagcctgtat ttccatattt aaatcctaga 1440

aaatgtggag tccccatgac tctgtgctca ccaagctctt ga 1482